(19) Europäisches Patentamt
European Patent Office
Office européen des brevets

(11) Veröffentlichungsnummer: **0 083 335**
**B1**

(12) # EUROPÄISCHE PATENTSCHRIFT

(45) Veröffentlichungstag der Patentschrift: **12.08.87**

(51) Int. Cl.⁴: **C 07 D 307/93**, C 07 D 311/94, C 07 B 57/00

(21) Anmeldenummer: **82890185.0**

(22) Anmeldetag: **17.12.82**

(54) **Chirale, optisch aktive Verbindungen, Verfahren zu ihrer Herstellung und Verwendung dieser Verbindungen für den Schutz funktioneller -OH, -SH, -NH-Gruppen, zur Racemattrennung, zur Herstellung optisch aktiver Imidoesterhydrochloride sowie optisch aktiver Ester, zur Herstellung optisch angereicherter Alkohole und**

(30) Priorität: **18.12.81 AT 5453/81**

(43) Veröffentlichungstag der Anmeldung: **06.07.83 Patentblatt 83/27**

(45) Bekanntmachung des Hinweises auf die Patenterteilung: **12.08.87 Patentblatt 87/33**

(84) Benannte Vertragsstaaten: **AT BE CH DE FR GB IT LI NL SE**

(56) Entgegenhaltungen: **Die Akte enthält technische Angaben, die nach dem Eingang der Anmeldung eingereicht wurden und die nicht in dieser Patentschrift enthalten sind.**

(73) Patentinhaber: **Noe, Christian, Dipl.- Ing. Dr., Larochegasse 20/7, A-1130 Wien (AT)**

(72) Erfinder: **Noe, Christian, Dipl.- Ing. Dr., Larochegasse 20/7, A-1130 Wien (AT)**

(74) Vertreter: **Berger, Erhard, Dr., Siebensterngasse 39 Postfach 306, A-1071 Wien (AT)**

**EP 0 083 335 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Chirale, enantiomerenreine Verbindungen, Verfahren zu ihrer Herstellung und Verwendung dieser Verbindungen für den Schutz funktioneller -OH, -SH, -NH-Gruppen, zur Racemattrennung, zur Herstellung optisch aktiver Imidoesterhydrochloride sowie optisch aktiver Ester, zur Herstellung optisch angereicherter Alkohole und zur Herstellung optisch aktiver Verbindungen durch asymmetrische Induktion.

Die vorliegende Erfindung betrifft neue chirale, enantiomerenreine Verbindungen der allgemeinen Formel

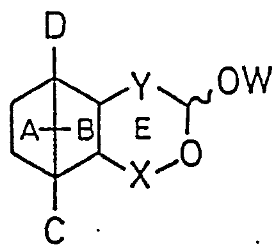

(I),

worin an ein Bicyclo[2.2.1]heptan-Ringsystem, in welchem A, B, C, D die Bedeutung H oder Merhyl in beliebiger Kombination haben, vorzugsweise ein Bornan-Ringsystem, ein 5- oder 6-gliedriger Lactolring (E) stereospezifisch anneliert ist, worin X und Y jeweils einen Rest $(CR^1R^2)_n$ mit n = 0 bis 2 und $R^1$ und $R^2$ Wasserstoff, Niederalkyl oder Aryl in beliebiger Kombination bedeuten, soweit die anomere Selektivität bei Acetalisierungsreaktionen nicht beeinträchtigt wird, und W Wasserstoff, substituiertes oder unsubstituiertes Alkyl, Cycloalkyl oder das Ringsystem selbst, insbesondere H, das Ringsystem selbst oder Niederalkyl bedeutet, bzw. für den Fall als W Wasserstoff ist deren Anhydroverbindungen der allgemeinen Formel

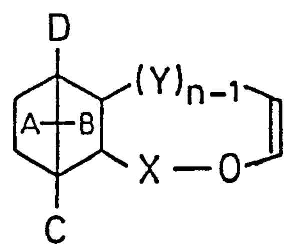

(V),

worin A, B, C, D, X und Y die gleiche Bedeutung wie in der hydrarisierten Form haben und n 1 oder 2 bedeuten, Verfahren zu ihrer Herstellung und die Verwendung dieser neuen Verbindungen für den Schutz funktioneller -OH, -SH, -NH-Gruppen zur Racemattrennung, zur Herstellung optisch aktiver Imidoesterhydrochloride sowie optisch aktiver Ester, zur Herstellung optisch angereicherter Alkohole und zur Herstellung optisch aktiver Verbindungen durch asymmetrische Induktion.

Die bevorzugten Verbindungen gemäß der Erfindung sind chirale, enantiomerenreine Verbindungen ((R)-Enantiomer bzw. (S)-Enantiomer) der allgemeinen Formel

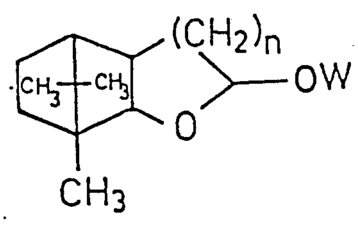

(Ia),

worin W die oben angegebene Bedeutung hat und n 1 oder 2 bedeuten, bzw. für den Fall als W Wassersroff ist, deren Anhydroverbindungen der allgemeinen Formel

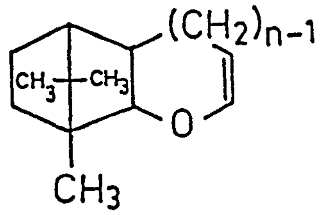

(Va),

worin n 1 oder 2 bedeuten.

Besonders bevorzugte Verbindungen gemäß der Erfindung sind chirale, enanriomerenreine (R)-Enantiomere und (S)-Enantiomere der allgemeinen Formel

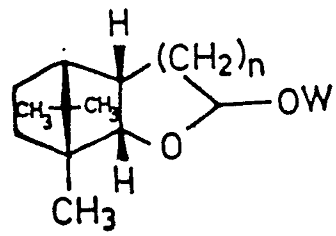

(Ib),

worin W die oben angegebene Bedeurung hat und n 1 oder 2 bedeuten, bzw. für den Fall, als W Wassersroff ist, deren Anhydroverbindungen der allgemeinen Formel

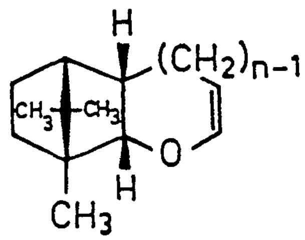

(Vb),

worin n die angegebene Bedeutung har.

Ein beträchrlicher Nachteil der Terrahydropyranyl- und einer Reihe weiterer bekannter acetalischer Schutzgruppen ist das Auftreren von Diastereomeren bei der Umsetzung mir chiralen Verbindungen und die damit verbundene Komplizierung der Produktisolierung und weiterer Reaktionen.

Die neuen chiralen enantiomerenreinen Verbindungen gemäß der Erfindung reagieren aufgrund ihrer Struktur in vielen Fällen mit hoher anomerer Selektivität mit Alkoholen, Cyanhydrinen, Thiolen, Aminen usw.

Abgesehen von der Funktion als Schutzgruppe wird durch diese anomere Selekrivität das Aufrreten von Diastereomeren bei der Umsetzung mit enantiomer einheitlichen chiralen Verbindungen vermieden, bei der Umsetzung mit racemischen Verbindungen kommt es zur Bildung eines Diastereomerenpaares, wodurch günstige Voraussetzungen für eine Racematspaltung durch Diastereomerentrennung gegeben sind. Bei der Reaktion mit geeigneten prochiralen Verbindungen werden Verbindungen der allgemeinen Formel III erhalten, welche günstige Voraussetzungen für Reaktionen mit asymmetrischer Induktion bieten können. Die für die erfindungsgemäße Anwendung der neuen Verbindungen der allgemeinen Formel I gemäß dieser Erfindung wesentliche Reaktion sei durch das folgende Schema 1 illustriert:

Schema 1

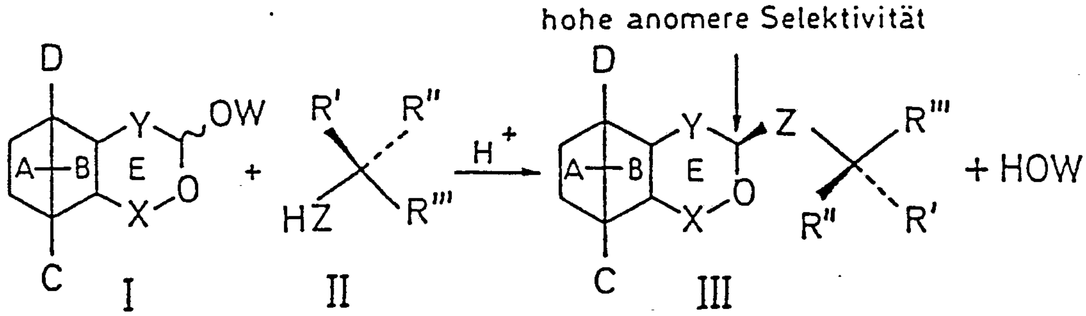


I II III

Die allgemeine Formel 1 bezeichnet das abgesehen vom Rest OW enantiomer einheitliche (optisch aktive) Edukt für die Umsetzungen mit einer Verbindung der allgemeinen Formel II, wobei an ein Bicyclo[2.2.1]heptan-Ringsystem, in welchem A, B, C und D die Bedeutung H und Methyl in beliebiger Kombination besitzen, vorzugsweise ein Bornan-Ringsystem (leicht erhältlich aus D(+)oder L(-)-Campher), ein weiterer 5- oder 6-gliedriger Lactolring (E) stereospezifisch anelliert ist, worin X und Y jeweils einen Rest $(CR^1R^2)_n$ mit n = 0 bis 2 und $R^1$ und $R^2$ Wasserstoff, Niederalkyl oder Aryl in beliebiger Kombination bedeuten, soweit die anomere Selektivität bei Acetalisierungsreaktionen nicht beeinträchtigt wird, und W die Bedeutung H sowie die in der Folge erläuterten Bedeutungen hat. Die ursprüngliche Konfiguration des Restes OW in der allgemeinen Formel 1 ist nicht wesentlich, da Acetalisierungsreaktionen über ein Carbeniumion der allgemeinen Formel IV verlaufen. Aus dem gleichen Grund kommt auch dem Substituenten W nur untergeordnete Bedeutung zu.

Schema 2

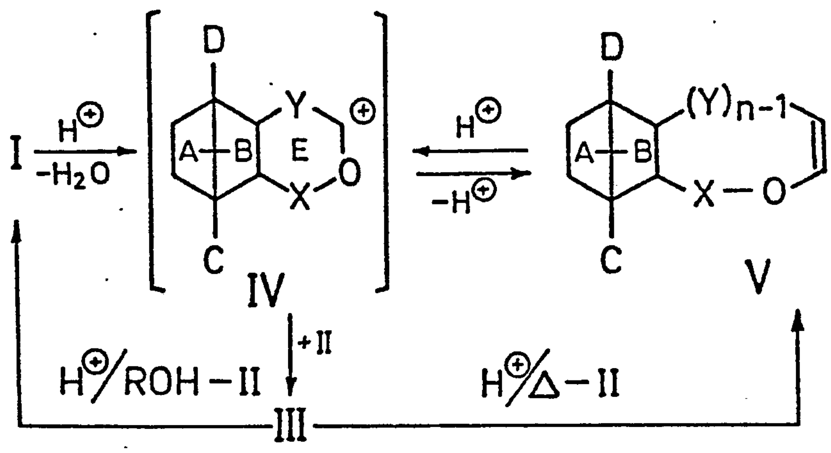


Aus diesem Grunde sind auch die beiden dehydratisierten Formen, nämlich die Verbindung der allgemeinen Formel I, in welcher W das Ringsystem selbst bedeutet (formal: Lactol - 1/2 $H_2O$), und welche durch säurekatalysierte Selbstkondensation des Lactols gebildet wird, sowie der Enolether der allgemeinen Formel V (formal: Lactol - $H_2O$), welcher aus dem Lactol unter Einwirkung von wasserabspaltenden Mitteln oder aus Verbindungen der allgemeinen Formel III (Z = O) bei der Vakuumdestillation in Gegenwart von Säurespuren durch Abspaltung der Verbindung der allgemeinen Formel II entsteht, als Verbindungen im Sinne der Erfindung aufzufassen.

Da im Sinne einer Umacetalisierung, welche ebenfalls über das Carbeniumion der allgemeinen Formel IV verläuft, Verbindungen der allgemeinen Formel III in manchen Fällen günstig aus anderen Verbindungen der allgemeinen Formel III hergestellt werden können, bedeutet W auch substituiertes oder unsubstituiertes Alkyl oder Cycloalkyl, vorzugsweise Niederalkyl.

Optisch aktive Verbindungen der allgemeinen Formel 1 (W = H) lassen sich leicht durch Reduktion aus entsprechenden Lactonen der allgemeinen Formel VI herstellen, wobei diese Reduktion vorzugsweise mit Hydridreagentien erfolgt.

Schema 3

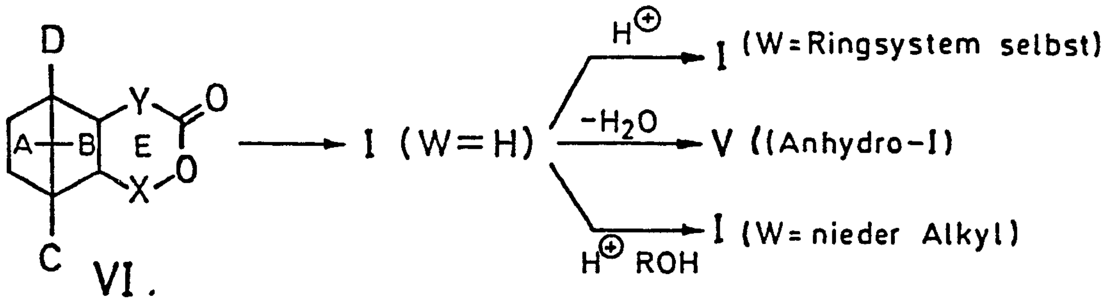


VI.

Aus Verbindungen der allgemeinen Formel I (W = H) lassen sich durch Behandeln mit katalytischen Mengen starker Säure in inertem Lösungsmittel die selbstkondensierten Verbindungen der allgemeinen Formel 1 (W=Ringsystem selbst) oder durch Behandeln mit wasserentziehenden Mitteln, vorzugsweise anorganisches Säurechlorid/Base, die Enolether der allgemeinen Formel V oder durch Behandeln mit katalytischen Säuremengen in Gegenwart von acyclischem oder cyclischem Alkohol, vorzugsweise niederem Alkohol, Acetale der allgemeiner Formel 1 (W=substituiertes oder unsubstituiertes Alkyl oder Cycloalkyl vorzugsweise Niederalkyl) gewinnen, welche besonders in Hinblick auf wiederholte Acetalisierungsreaktionen gut geeignete Schutzgruppenreagentien darstellen. Als weitere Verfahren zur Herstellung optisch aktiver Verbindungen der allgemeinen Formel I sind die selektive Reduktion und anschließende Cyclisierung von Oxosäurederivaten, die partielle Oxidation von Diolen, die Addition von Wasser an Enolether der allgemeinen Formel V, die photochemische Ringerweiterung anellierter Cyclobutanone, sowie Cycloadditionsreaktionen zu nennen. Die Gewinnung optisch aktiver Verbindungen der allgemeinen Formel I durch Racematspaltung aus racemischen Verbindungen der allgemeinen Formel I stellt besonders für die in ihrer racemischen Form leicht zugänglichen Norbornanderivate der allgemeinen Formel I (A, B, C, D = H) ein weiteres Herstellungsverfahren dar. (racemische Ausgangsprodukte der allgemeinen Formel I (A, B, C, D = H) siehe: R.D.Miller, D.L.Dolce, V.Y.Merrit, Tetrahedron Lett. 1974 (37), 3347-50; G.B.Ohloff, W.D.Skorianetz, Ger. Offen. 28 26 302, 15.6.1978; R.L. Snowden, P.Sonnay, G.Ohloff, Helv. Chim. Acta 64, 25-32, 1981).

Verbindungen der allgemeinen Formel V sind außer durch Eliminierungsreaktionen von Verbindungen der allgemeinen Formel I oder II ebenfalls durch Cycloadditionsreaktionen gut zugänglich.

Formel II in vorangehendem Reaktionsschema bedeutet im Sinne der Erfindung die mit einer Verbindung der allgemeinen Formel I umzusetzende bzw. zu schützende Verbindung. Voraussetzung für den Einsatz als Schutzgruppenreagens ist lediglich das Vorliegen einer -OH, -NH- oder -SH Funktion in der Verbindung der allgemeinen Formel II (Z = O, S, NH), welche genügend Reaktivität aufweist, um mit einer Verbindung der allgemeinen Formel I zu reagieren; bei der Verwendung zur asymmetrischen Induktion darüberhinaus eine geeignete prochirale Gruppierung; und beim Einsatz als Reagenz zur Racemattrennung ein chirales Zentrum möglichst in Nachbarschaft zur -OH, -NH- oder -SH Gruppe. Die Reste R', R'', und R''' bedeuten H, sowie voneinander unabhängige oder miteinander verknüpfte substituierte oder unsubstituierte Alkyl-, Aryl-, Heteroaryl-, Carbonyl-, Carboxyl- oder Nitrilgruppen, welche den genannten Bedingungen Genüge tun. Für Acylverbindungen bedeutet CR'R'' auch

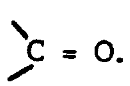

Vorzugsweise bedeuten die Reste R', R'' und R''' Wasserstoff, Alkyl, 1-Hydroxyalkyl, 1-Oxoalkyl, Aryl, substituiertes Aryl, Heteroaryl, Aroyl, Heteroaroyl, Carboxyl, Alkoxycarbonyl, Aryloxycarbonyl, Aminocarbonyl und Nitril in beliebigen Kombinationen, sofern diese die Reaktivität von II mit Verbindungen I bzw. V nicht beschränken. Für cyclische Strukturen bedeuten R' und R'' vorzugsweise $-(CR^1R^2)_n-$, worin n = 3 bis 7 ist und $R^1$ und $R^2$ Alkyl und Aryl bedeuten, sowie $-O-Aryl-(CR^1R^2)_n-$, worin n = 2 bis 4 ist und $R^1$ und $R^2$ Alkyl oder Aryl in beliebiger Kombination bedeuten.

Verbindungen der allgemeinen Formel III lassen sich durch säurekatalysierte Reaktion von Verbindungen der allgemeinen Formel I mit Verbindungen der allgemeinen Formel II herstellen. (Verbindungen I (W = Ringsystem selbst) reagieren unter den im experimentellen Teil angegebenen Bedingungen nicht mit Aminen). Über den Schutz der funktionellen Gruppe in Verbindungen der allgemeinen Formel II hinaus besteht beim Einsatz racemischer Verbindungen der allgemeinen Formel II in vielen Fällen die Möglichkeit zur Racematspaltung durch Trennung der diastereomeren Verbindungen der allgemeinen Formel III (vorzugsweise Säulenchromatographie oder Kristallisation). Nach erfolgter Diastereomerentrennung und allenfalls durchgeführten weiteren Reaktionen mit den getrennten Verbindungen der allgemeinen Formel III kann unter Säurekatalyse die Schutzgruppe, z. B. als Dimeres der allgemeinen Formel I (W = Ringsystem selbst) oder

Methylacetal der allgemeinen Formel I (W = Methyl) abgespalten und ein reines Enantiomer der allgemeinen Formel II gemäß dem folgenden Reaktionsschema gewonnen werden.

Schema 4

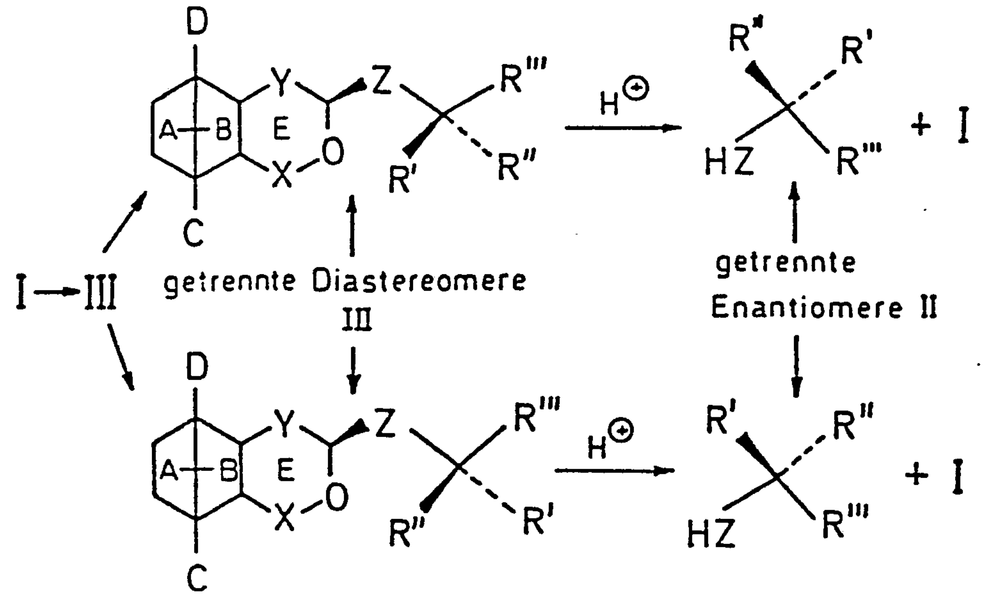


Aus geschützten Cyanhydrinen können auf diese Weise nach erfolgter Diastereomerentrennung enantiomer einheitliche Imidoesterhydrochloride gewonnen werden. Bei der Acetalisierung racemischer Alkohole der allgemeinen Formel II (Z = O) ergibt sich unter bestimmten strukturellen Voraussetzungen (z. B. R' = H, R'' oder R''' = Aryl) eine bevorzugte Reaktion eines Enantiomeren und damit die Möglichkeit optisch angereicherte Alkohole zu erhalten.

Bei der Umwandlung eines prochiralen Restes ZCR' R''R''' von Verbindungen der allgemeinen Formel III in einen chiralen Rest kommt es in einer Reihe von Fällen zur asymmetrischen Induktion, wobei bei der Aufarbeitung nicht nur der Schutzgruppencharakter, sondern auch die gute Trenbarkeit von diastereomeren Verbindungen der allgemeinen Formel III günstige Synthesemöglichkeiten für chirale Verbindungen schaffen, wodurch auch asymmetrische Reaktionen mit nicht sehr hohen Enantiomerenüberschüssen (EE) präparativ interessant werden. Stereospezifische Reaktionen, welche in diesem Zusammenhang zur Anwendung kommen können, sind z. B. Alkylierung oder Alkylierung nach Deprotonierung, Grignard-Reaktion oder Reaktionen mit Organo-Lithium-Verbindungen. Diese Anwendungsvariante wird anhand der Alkylierung von Mercaptoessigsäure und deren Ester (in der allgemeinen Formel II bedeuten: Z = S, R', R'' = H, R''' = COOR) illustriert.

Die folgenden Beispiele sollen die gegenständliche Erfindung erläutern, wobei sich die Beispiele 1 und 2 auf die Herstellung der neuen chiralen enantiomerenreine Verbindungen beziehen, während die Beispiele 3 bis 12 die Verwendung der neuen Verbindungen gemäß der Erfindung veranschaulichen. In allen Beispielen werden die Verbindungen der allgemeinen Formel I bzw. V durch Verbindungen der allgemeinen Formel Ib bzw. Vb, welche rechtsdrehende Enantiomere mit A, B, C = Methyl, D = H, und endo-cis-anelliertem Lactolring (E), worin in der Gruppe X:n = 0 und Y = $(CH_2)_{1-2}$ sind, repräsentiert. Für diese Schutzgruppenreste wurden die Abkürzungen MBF (n = 1) und MBP (n = 2) gewählt.

In allen angegebenen Beispielen werden rechtsdrehende Verbindungen als Ausgangsmaterial angegeben. Die linksdrehenden Enantiomeren, welche natürlich identische physikalische Daten aufweisen, werden ohne irgendwelche Änderungen im Herstellungsverfahren beim Einsatz linksdrehender Ausgangsprodukte erhalten.

**Beispiel 1**: MBF-Schutzgruppenreagentien

a) <u>MBF-OH durch Reduktion</u>

[2S-(2α,3aα,4α,7α,7aα)]-2,3,3a,4,5,6,7,7a-Octahydro-7,8,8-trimethyl-4,7-methanobenzofuran-2-ol (MBF-OH) (n = 1, W = H):

In eine Lösung von 11,6 g [3aS-(3aα,4α,7α,7aα)]-3a,4,5,6,7,7a-Hexahydro-7,8,8-trimethyl-4,7-

methanolbenzofuran-2(3H)-on in 50 ml wasserfreiem Toluol werden unter Rühren bei -40° C 55 ml einer 20 proz. Lösung von Diisobutylaluminiumhydrid in n-Hexan zugetropft. Nach 2 Stunden Rühren werden bei -40° C 80 ml Ether und 30 ml Wasser zugegeben. Bei Raumtemperatur wird bis zur Auflösung des Aluminiumhydroxid-Niederschlages 2N NaOH zugesetzt, die organische Phase abgetrennt und die wäßrige Phase mit Ether extrahiert. Die vereinigten organischen Lösungen werden mit Wasser gewaschen, getrocknet und eingedampft, Ausb.: 11,5 g (98 %) farbloses Öl, das bei Temperaturen unter 5° C kristallisiert, Sdp. 120° C/0,005 Torr (Luftbad).

$^1$H-NMR ($CDCl_3$): δ = 5,55-5,75 (dd; 1H); 4,40 (d, I = 9 Hz; 1H); 4,0 (s, 1H, austauschbar mit $D_2O$); 2,7-3,2 (m, 1H); 1,1-2,1 (m, 7H); 1,00/0,93/0,88 (3s, 9H). Gehalt an β(endo)-Anomerem ~7 %.

Das Ausgangsprodukt kann folgendermaßen hergestellt werden:

[3aS-(3aα,4α,7α,7aα)]-3a,4,5,6,7,7a-Hexahydro-7,8,8-trimethyl-4,7-methanobenzofuran-2(3H)-on: In eine Lösung von 51 g Campheressigsäure in 750 ml Ethanol und 121 ml 2N NaOH werden bei Raumtemperatur 9,2 g $NaBH_4$ eingetragen. Es wird 16 h bei 50° C gerührt, im Vakuum auf 450 ml eingeengt und mit Wasser auf 750 ml verdünnt. Es wird mit 10 proz. Schwefelsäure angesäuert, mehrmals mit Petrolether/Ether 9 : 1 extrahiert, die organische Phase mit verdünnter Schwefelsäure gewaschen, getrocknet und eingedampft. Der Rückstand wird in Petrolether/Ether 9 : 1 aufgenommen und mit einer halbgesättigten Natriumbicarbonat-Lösung, welcher 2,5 % Methanol zugesetzt werden, ausgeschüttelt. Die organische Phase wird getrocknet, eingedampft und der Rückstand aus Petrolether/Ether 9 : 1 umkristallisiert.

Ausb. 25,5 g (55 %) farblose Kristalle, Schmp. 48-50° C.

b) [2R-(2α(2'R*,3'aS*,4'R*,7'R*,7'aS*),3aα,4α,7α,7aα]-2,2'-Oxybis [2,3,3a,4,5,6,7,7a-octahydro-7,8,8-trimethyl-4,7-methanobenzofuran] (MBF-O-MBF): (n = 1, W = MBF)

1) Eine Lösung von 1 g MBF-OH in 5 ml Ether wird mit 5 Tropfen Chlorwasserstoff-gesättigtem Ether versetzt und 3 Stunden uber Molekularsieb 4 Å stehen gelassen, Es wird vom Molekularsieb dekantiert, der Ether verdunsten gelassen und der kristalline Rückstand aus Petrolether umkristallisiert.

Ausb. 0,83 g (87 %) farblose Kristalle, Schmp. 151-152° C; $[\alpha]_D^{20}$ = +193° (c = 2,25 in THF).

$^1$H-NMR ($CDCl_3$): δ = 5,64/5,62/5,58/5,56 (dd 1H); 4,25 (d, I = 9,5 Hz, 1H); 2,7-3,15 (m, 1H); 1,0-2,1 (m, 7H); 098/0,90/0,87 (3s, 9H).

2) Eine Lösung von 1,05 g O-MBF-Methanol (n = 1, W = $CH_3$) in 5 ml Ether wird mit 5 Tropfen Chlorwasserstoff-gesättigtem Ether versetzt und verdunsten gelassen. Der kristalline Rückstand wird aus Petrolether umkristallisiert. Ausb. 0,77 g (80 %) farblose Kristalle, Schmp. 150-152° C.

c) [2S-(2α,3aα,4α,7α,7aα)]-2,3,3a,4,5,6,7,7a-Octahydro-2-methoxy-7,8,8-trimethyl-4,7-methanobenzofuran (O-MBF'-Methanol): (n = 1, W = $CH^3$) : Eine Lösung von 1,0 g MBF-OH und 0,33 g Methanol in 20 ml Ether/Petrolether 1 : 1 wird mit ca. 10 mg p-Toluolsulfonsäure und einigen Körnern Molekularsieb 4 Å versetzt und 1 Stunde bei Raumtemperatur gerührt. Die Lösung wird mit Ether verdünnt und mit Natriumbicarbonat-Lösung extrahiert. Die organische Phase wird getrocknet und eingedampft. Ausb. 1,06 g (99 %) farbloses Öl, Sdp. 80° C/0,02 Torr (Luftbad).

$^1$H-NMR ($CDCl_3$): δ = 5,17/5,14/5,10/5,07 (dd, 1H); 4,2 (d, I = 9 Hz, 1H); 3,3 (s, 3H); 2,7-3,1 (m, 1H); 1,1-2,2 (m,7H), 0,97/0,91/0,88 (3s, 9H).

Gehalt an β/endo-Anomerem ~ 7 %.

d) [2S-(2α,3aα,4α,7α,7aα)]-2,3,3a,4,5,6,7,7a-Octahydro-2-ethoxy-7,8,8-trimethyl-4,7-methanobenzofuran (O-MBF-Ethanol): (n = 1, W = Et)

Synthese analog c) mit Ethanol: farbloses Öl, Sdp. 90° C/0,02 Torr (Luftbad).

$^1$H-NMR ($CDCl_3$): δ = 5,34/5,32/5,29/5,27 (dd, 1H); 4,27 (d, I = 9,3 Hz, 1H); 3,45 (q, I = 8 Hz, 2H), 2,7-3,1 (m, 1H); 1,4-1,9 (m, 8H); 1,1-1,25 (m, 3H); 0,97/0,90/0,86 (3s, 9H). Gehalt an β/endo-Anomerem ~ 7 %.

e) [2S-(2α,3aα,4α,7α,7aα)]-2,3,3a,4,5,6,7,7a-Octahydro-2-isopropoxy-7,8,8-trimethyl-4,7-methanobenzofuran (O-MBF-Isopropanol): (n = 1, W = $CH(CH_3)_2$)

Synthese analog c) mit Isopropanol: farbloses Öl, Sdp. 90° C/0,02 Torr (Luftbad).

$^1$H-NMR ($CDCl_3$): δ = 5,44/5,42/5,39/5,37 (dd, 1H); 4,26 (d, I = 9,5 Hz, 1H); 3,7-4,2 (m, 1H); 2,5-3,0 (m, 1H); 1,0-1,9 (m, 13H); 0,97/0,90/0,85 (3s, 9H).

Gehalt an β/endo-Anomerem ~ 7 %.

f) [3aR-(3aα,4α,7α,7aα)]-3a,4,5,6,7,7a-Hexahydro-7,8,8-trimethyl-4,7-methanobenzofuran: (V; Anhydro-MBF)

Eine Lösung von 0,50 g MBF-OH in 5 ml Thionylchlorid wird 2 Stunden bei Raumtemperatur gerührt, das überschüssige Thionylchlorid im Vakuum abdestilliert. Der Rückstand wird in 10 ml wasserfreiem Ether mit 1 ml Triethylamin 2 Stunden bei 50° C gerührt, filtriert, der Ether im Vakuum abdestilliert und das Rohprodukt durch SC (Eluens: Petrolether/Ether 100 : 1, triethylaminimprägniertes Kieselgel) gereinigt. Ausb. 0,32 g (71 %) Anhydro-MBF; farbloses Öl.

$^1$H-NMR ($CDCl_3$): δ = 6,36/6,34/6,33/6,31 (dd, 1H); 4,72 (t, I = 2,8 Hz, 1H); 4,67/4,65/4,54/4,52 (dd, 1H); 3,22-3,53 (m, 1H); 1,0-1,8 (m, 5H); 0,94/0,92/0,91 (3s, 9H).

**Beispiel 2**: MBP-Schutzgruppenreagentien

a) MBP-OH durch Reduktion
[2S-(2α,4aα,5α,8α,8aα)]-3,4,4a,5,6,7,8,8a-Octahydro-8,9,9-trimethyl-5,8-methano-2H-1-benzopyran-2-ol (MBP-OH): (n = 2, W = H)
Synthese analog MBF-OH aus [4aS-(4aα,5α,8α,8aα)]-3,4,4a,5,6,7,8,8a-Octahydro-8,9,9-trimethyl-5,8-methano-2H-1-benzopyran-2-on.
Es wurden farblose Kristalle erhalten, Schmp. 98-101°C.
$^{1}H$-NMR ($CDCl_3$): δ = 5,35 (t, I = 6 Hz, 1H); 3,95 (d, I = 10 Hz, 1H); 3,5 (s, 1H); 2,35-1,0 (m, 10H); 0,90 (s, 6H); 0,86 (s, 3H).
b) [2R-2α(2'R*,4a'S*,5'R*,8'R*,8a'S*)4aα,5α,8α,8aα]-2,2'-Oxybis-[3,4,4a,5,6,7,8,8a-octahydro-8,9,9-trimethyl-5,8-methano-2H-1-benzopyran] (MBP-O-MBP): (n = 2, W = MBP)
Synthese analog MBF-O-MBF aus MBP-OH: farblose Kristalle, Schmp. 123-124°C; $[\alpha]_D^{26}$ = +167° (c = 2,0 in n-Hexan);
$^{1}H$-NMR ($CDCl^3$): δ = 5,28 (t, I = 6,5 Hz, 1H); 3,73 (d, I = 9,5 Hz, 1H); 1,7-2,2 (m, 3H); 1,0-1,6 (m, 7H); 0,89 (s, 6H); 0,83 (s, 3H).
c) [2S-(2α,4aα,5α,8α,8aα)]-3,4,4a,5,6,7,8,8a-Octahydro-2-methoxy-8,9,9-trimethyl-5,8-methano-2H-1-benzopyran (O-MBP-Methanol): (n = 2, W = $CH_3$)
Synthese analog O-MBF-Methanol aus MBP-OH;
farbloses Öl, Sdp. 80°C/0,02 Torr (Luftbad);
$^{1}H$-NMR ($CDCl_3$): δ = 4,77 (t, I = 8 Hz, 1H); 3,71 (d, I = 10 Hz, 1H); 3,32 (s, 3H); 1,13-2,25 (m, 10 H); 0,90 (s, 6H); 0,87 (s, 3H).

**Beispiel 3**: Verwendung als Schutzgruppe

Acetalisierung (allgemeine Arbeitsvorschrift): Eine 5 proz. Lösung von MBF-OH, MBF-O-MBF bzw. MBP-OH, MBP-O-MBP oder Anhydro-MBF und einer Verbindung der Formel II (1,1 - 4 Äquivalente) in wasserfreiem Ether (oder Dichlormethan, THF, Chloroform, petrolether/Ether) wird nach Zugabe katalytischer Mengen p-Toluolsulfonsäure (oder Chlorwasserstoff-gesättigten Ethers) und einiger Körner Molekularsieb 4 Å 2 Stunden bei Raumtemperatur gerührt. Es wird festes Natriumbicarbonat und Natriumsulfat zugegeben, filtriert und eingedampft. Der Ruckstand wird, wenn erforderlich, durch Säulenchromatographie über triethylaminimprägniertes Kieselgel aufgetrennt. Nach dieser allgemeinen Vorschrift werden auch die in den nächsten Beispielen zur Racemattrennung verwendeten Verbindungen hergestellt.
Beispiele mit nicht chiralen Verbindungen II:
a) [2S-(2α,3aα,4α,7α,7aα)]-(2,3,3a,4,5,6,7a-Octahydro-7,8,8-trimethyl-4,7-methanobenzofuran-2-yl)oxy]acetonitril (O-MBF-Hydroxyacetonitril) (Z = O, R' = R'' = H, R''' = CN):
Aus 2,0 g MBF-O-MBF und 0,80 g Hydroxyacetonitril in Dichlormethan werden unter p-Toluolsulfonsäurekatalyse nach säulenchromatographischer Reinigung 2,0 g (82 %) farblose Kristalle erhalten; Schmp. 33-34°C; $[\alpha]_D^{20}$ = +188° (in n-Hexan);
$^{1}H$-NMR ($CDCl_3$): δ = 5,40 (d, I = 4,5 Hz, 1H); 4,1-4,35 (m, 3H); 2,7-3,1 (m, 1H); 1,1-2,0 (m, 7H); 0,96/0,89/0,84 (3s, 9H).
b) [2S-(2α,3aα,4α,7α,7aα)]-2,3,3a,4,5,6,7,7a-Octahydro-2-(cyclohexyloxy)-7,8,8-trimethyl-4,7-methanobenzofuran (O-MBF-Cyclohexanol) (Z = O, R' = H, R''R''' = $-(CH_2)_5-$):
Nach allgemeiner Arbeitsvorschrift; farbloses Öl, Sdp. 90°C/0,02 Torr (Luftbad);
$^{1}H$-NMR ($CDCl_3$): δ = 5,49/5,47/5,44/5,42 (dd, 1H); 4,26 (d, I = 9,4 Hz, 1H); 3,3-3,8 (m, 1H); 2,7-3,1 (m, 1H); 1,1-2,0 (m, 17H); 0,96/0,89/0,85 (3s, 9H).
c) [2S-(2α,3aβ,4β,7β,7aβ)]-[(2,3,3a,4,5,6,7,7a-Octahydro-7,8,8-trimethyl-4,7-methanobenzofuran-2-yl)amino]essigsäure-t-butylester-hydrochlorid (N-MBF-Aminoessigsäure-t-butylester-hydrochlorid)

$$(Z = \overset{\oplus}{N}H_2Cl, R'=R''=H, R'''= COO\text{-}t\text{-}Bu):$$

Eine Lösung von 1,0 g MBF-OH und 0,70 g Aminoessigsäure-t-butylester in Dichlormethan wird mit etwas Molekularsieb 4 Å und katalytischen Mengen p-Toluolsulfonsäure versetzt. Nach 2 Stunden wird die Lösung mit HCl-gesättigtem trockenen Ether versetzt und eingedampft. Der Rückstand wird aus Dichlorme than/Ether umkristallisiert. Ausb. 1,29 g (73 %) farblose Kristalle, Schmp. 155°C; $[\alpha]_D^{20}$ = +75° (c = 1,2 in Dichlormethan);
$^{1}H$-NMR ($CDCl_3$): δ = 9,87 (s, 2H); 5,28 (d, 1H); 4,53 (d, I = 10 Hz, 1H); 3,72 (s, 2H); 3,13-3,44 (m, 1H); 2,32-2,72 (m, 2H); 1,0-1,78 (m, 14H); 0,99/0,90/0,84 (3s, 9H).

**Beispiel 4**: Trennung von racemischen Alkoholen

Dieses Verfahren gliedert sich in 3 Schritte:
1. Acetalisierung
2. Trennung der Diastereomeren
3. Abspaltung der Schutzgruppe

a) Acetalisierung von racemischem 1-Phenylethanol mit der MBF-Schutzgruppe: Acetalisierung: 1,0 g MBF-O-MBF und 2,61 g rac. 1-Phenylethanol nach der allgemeinen Vorschrift in Beispiel 3. Zunächst wird überschüssiges 1-Phenylethanol durch SC abgetrennt (Eluens: Petrolether/Ether 20:1). Die Ausbeute an O-MBF-1-Phenylethanol (Diastereomerengemisch (R)/(S) ~ 2/1 laut $^1$H-NMR) beträgt 1,41 g (38 %) farbloses Öl, Sdp. 120° C/0,005 Torr (Luftbad).

Trennung: Bei der folgenden SC-Trennung über triethylaminimprägniertes Kieselgel (Eluens: Petrolether/Ether 20:1) werden 895 mg (56 %) (R)-O-MBF-1-Phenylethanol und 345 mg (22 %) (S)-O-MBF-1-Phenylethanol erhalten. (R)-O-MBF-1-Phenylethanol [2S-(2α(S*),3aα,4α,7α,7aα)]-2,3,3a,4,5,6,7,7a-Octahydro-7,8,8-trimethyl-2-(1-phenylethoxy)-4,7-methanobenzofuran) (Z = O, R' = Phenyl, R'' = H, R''' = $CH_3$): farblose Kristalle, Schmp. 72-74° C (Petrolether); $[\alpha]_D^{21}$ = +221 (c = 8,3 in Benzol); $^1$H-NMR ($CDCl_3$): δ = 7,25 (s, 5H); 5,12 (t, I = 3 Hz, 1H); 4,78 (q, I = 6,6 Hz, 1H); 4,32 (d, I = 9,1 Hz, 1H); 2,7-3,1 (m, 1H); 1,0-1,8 (m, 10H); 0,95 (s, 3H); 0,86 (s, 6H). (S)-O-MBF-1-Phenylethanol ([2S-(2α-(R*,3aα,4α,7α,7aα)]-2,3,3a,4,5,6,7,7a-Octahydro-7,8,8-trimethyl-2-(1-phenylethoxy)-4,7-methanobenzofuran) (Z = O, R' = H, R'' = Phenyl, R''' = $CH_3$): farbloses Öl; $[\alpha]_D^{21}$ = +21,4° (c = 12 in Benzol); $^1$H-NMR ($CDCl_3$): δ = 7,30 (s, 5H); 5,35/5,50/5,48/5,45 (dd, 1H); 4,73 (q, I = 6,5 Hz, 1H); 3,95 (d, I = 9 Hz, 1H); 2,7-3,1 (m, 1H); 1,0-2,0 (m, 10H); 0,88 (s, 6H); 0,72 (s, 3H).

b) Trennung von rac. 1-Phenylethanol mit Anydro-MBF (V): 1,00 g Anhydro-MBF und 2,74 g rac. 1-Phenylethanol nach der Vorschrift wie in Beispiel 3) ergeben 1,48 g (88 %) O-MBF-1-Phenylethanol Diastereomerengemisch (Diastereomerenverhältnis wie in Beispiel 4a), welches wie in 4a angegeben säulenchromatographiert wird.

Abspaltung der Schutzgruppe:

Allgemeine Arbeitsvorschrift zur Methanolyse: 1 mmol Acetal in 10 ml MeOH wird mit einer Spatelspitze p-Toluolsulfonsäure versetzt und 3 Stunden stehengelassen, Es wird mit 0,5 g festem Natriumbicarbonat und Natriumsulfat versetzt, filtriert, eingedampft und der Rückstand über Kieselgel (nicht imprägniert) chromatographiert. Zuerst wird O-MBF-Methanol eluiert, welches nach der in Beispiel 1 angegebenen Vorschrift wieder zu MBF-O-MBF umgesetzt werden kann. Anschließend wird der chirale Alkohol eluiert, (R)-1-Phenylethanol (II: Z = O, R' = Phenyl, R'' = H, R''' = $CH_3$): Aus 308 mg (R)-O-MBF-1-Phenylethanol (SC: Eluens: Petrolether/Ether 100:1, dann Ether) werden 88 mg (72 %) (R)-1-Phenylethanol erhalten, Farbloses Öl, Sdp. 100° C/12 Torr (Luftbad). $[\alpha]_D^{23}$ = +52,4° (c = 3,72 in Benzol). (S)-1-phenylethanol (II: Z = O, R' = H, R'' = Phenyl, R''' = $CH_3$): Aus 299 mg (S)-O-MBF-1-Phenylethanol (SC: Eluens: Petrolether/Ether 20:1, dann Ether) werden 105 mg (86 %) (S)-1-Phenylethanol erhalten. Farbloses Öl, Sdp. 100° C/12 Torr (Luftbad). $[\alpha]_D^{20}$ = -46,2° (c = 2,5 in Chloroform).

c) Trennung von racemischem 1-Phenylethanol mit der MBP-Schutzgruppe:

Acetalisierung: 0,77 g MBP-O-MBP und 0,93 g rac. 1-Phenylethanol nach der allgemeinen Vorschrift in Beispiel 3. Nach Abtrennung des überschüssigen 1-Phenylethanols durch SC (Eluens: Petrolether/Ether 20:1) erhält man 1,07 g (90 %) O-MBP-1-Phenylethanol (Diastereomerengemisch: (R)/(S) ~ 2:1 laut $^1$H-NMR). Farbloses Öl.

Trennung: analog O-MBF-1-Phenylethanol ergibt 0,66 g (55 %) (R)-O-MBP-1-Phenylethanol und 0,34 g (29 %) (S)-O-MBP-1-Phenylethanol.

(R)-O-MBP-1-Phenylethanol ([2S-(2α(S*),4aα,5α,8α,8aα)]-3,4,4a,5,6,7,8,8a-Octahydro-8,9,9-trimethyl-2(1-phenylethoxy)-5,8-methano-2H-1-benzopyran) (Z = O, R' = Phenyl, R'' = H, R''' = $CH_3$): farbloses Öl, Sdp. 125° C/0,001 Torr (Luftbad). $[\alpha]_D^{23}$ = +188° (c = 2,25 in n-Hexan); $^1$H-NMR ($CDCl_3$): δ = 7,29 (s, 5H); 4,81 (m, 2H); 3,84 (d, I = 10 Hz, 1H); 1,6-2,3 (m, 3H); 1,0-1,6 (m, 10H); 0,92 (s, 3H); 0,90 (s, 6H).

(S)-O-MBP-1-Phenylethanol ([2S-(2α(R*),4aα,5α,8α,8aα)]-3,4,4a,5,6,7,8,8a-Octahydro-8,9,9-trimethyl-2(1-phenyl-ethoxy-5,8-methano-2H-1-benzopyran) (Z = O, R' = H, R'' = Phenyl, R''' = $CH_3$): farblose Kristalle Schmp. 85-86° C $[\alpha]_D^{22}$ = +52° (c = 0,50 in n-Hexan); $^1$H-NMR ($CDCl_3$): δ = 7,30 (s, 5H); 5,16 (t, 1H); 4,75 (q, 1H); 3,56 (dl = 10 Hz, 1H); 1,6-2,3 (m, 3H); 1,0-1,6 (m, 10H); 0,82/0,78/0,47 (3s, 9H), Abspaltung der Schutzgruppe: durch Methanolyse nach der allgemeinen Arbeitsvorschaft in Beispiel 4b) für O-MBF-1-Phenylethanol.

d) Trennung von racemischem 1-Phenylpropanol mit der MBF-Schutzgruppe: Acetalisierung: Aus 1,0 g MBF-OH und 2,75 g rac. 1-Phenylpropanol nach der allgemeinen Vorschrift in Beispiel 3. Zunächst wird überschüssiges 1-Phenylpropanol durch SC abgetrennt (Eluens: Petrolether/Ether 10:1), Die Ausbeute an Diastereomerengemisch ((R)/(S) ~ 3:5:1 laut $^1$H-NMR) beträgt 1,36 (85 %).

Trennung: Bei der folgenden SC Trennung über triethylaminimprägniertes Kieselgel (Eluens: Petrolether/Ether 20:1) werden 974 mg (61 %) (R)-O-MBF-1-Phenylpropanol und 249 mg (16 %) (S)-O-MBF-1-Phenylpropanol erhalten.

(R)-O-MBF-1-Phenylpropanol ([2S-(2α(S*),3aα,4α,7α,7aα)]-2,3,3a,4,5,6,7,7a-Octahydro-7,8,8-trimethyl-2-(1-phenylpropoxy)-4,7-methanobenzofuran) (Z = O, R' = Phenyl, R'' = H, R''' = Et): farbloses Öl, Sdp. 100° C/0,01 Torr (Luftbad);

$[\alpha]_D^{20}$ = +176° (c =1,9 in Hexan); $^1$H-NMR ($CDCl_3$): δ = 7,23 (s, 5H); 5,10 (tl = 3 Hz, 1H); 4,50 (t, I = 6,5 Hz, 1H); 4,30 (d, I = 9 Hz, 1H); 2,8-3,2 (m, 1H); 1,0-1,9 (m, 9H); 1,00 (s, 3H); 0,90 (s, 6H); 0,88 (t, I = 6,5 Hz, 3H). (S)-O-MBF-1-Phenylpropanol ([2S-(2α(R*),3aα,4α,7α,7aα)]-2,3,3a,4,5,6,7,7a-Octahydro-7,8,8-trimethyl-2-(1-phenylpropoxy)-4,7-methanobenzofuran) (Z = O, R' = H, R" = Phenyl, R"' = Et): farblose Kristalle, Schmp. 50-54°C (aus Petrolether); $[\alpha]_D^{20}$= +72,7°(c = 0,8 in Hexan); $^1$H-NMR ($CDCl_3$): δ = 7,23 (s, 5H); 5,42 (t, I = 3 Hz, 1H); 4,40 (t, I = 6 Hz, 1H); 3,78 (d, I = 9 Hz, 1H); 2,6-3,1 (m, 1H); 1,1-1,9 (m, 9H); 0,7-1,0 (m, 9H); 0,64 (s, 3H).

Abspaltung der Schutzgruppe durch Methanolyse nach der allgemeinen Arbeitsvorschrift in Beispiel 4b).

(R)-1-Phenyropanol (II: Z = O, R'= Phenyl, R" = H, R"' = Et):

Aus 551 mg (R)-O-MBF-1-Phenylpropanol (SC: Eluens: Petrolether/Ether 5:1, dann Ether) werden 199 mg (83 %) (R)-1-phenylpropanol als farbloses Öl erhalten; $[\alpha]_D^{20}$ = +41,7° (c = 2,3 in n-Hexan).

(S)-1-Phenylpropanol (II: Z = O, R' = H, R" = Phenyl, R"' = Et): Aus 65,7 mg (S)-O-MBF-1-Phenylpropanol (SC: Eluens: Petrolether/Ether 5:1, dann Ether) werden 21 mg (74 %) (S)-1-Phenylpropanol als farbloses Öl erhalten; $[\alpha]_D^{20}$ = -33,1° (c = 0,4 in Tetrahydrofuran).

e) Trennung von racemischem 2-Methyl-1-phenylpropanol mit der MBF-Schutzgruppe:

Acetalisierung: 2,0 g MBF-OH und 5,6 g rac. 2-Methyl-1-phenylpropanol nach der allgemeinen Vorschrift in Beispiel 3. Nach der Abtrennung von überschüssigem 2-Methyl-1-phenylpropanol durch SC (Eluens: Petrolether/Ether 20:1) werden 2,65 g (89 %) O-MBF-2-Methyl-1-phenylpropanol (Diastereomerengemisch: (R)/(S) ~ 2:1 laut $^1$H-NMR erhalten. Farbloses Öl, Sdp. 110°C/0,001 Torr (Luftbad). Trennung: säulenchromatographisch wie in Beispiel 4a) ergibt 1,51 g (51 %) (R)-O-MBF-2-Methyl-1-phenylpropanol und 0,85 g (29 %) (S)-O-MBF-2-Methyl-1-phenylpropanol.

(R)-O-MBF-2-Methyl-1-phenylpropanol ([2S-(2α(S*),3aα,4α,7α,7aα)]-2,3,3a,4,5,6,7,7a-Octahydro-7,8,8-trimethyl-2-(2-methyl-1-phenylpropoxy-4,7-methanobenzofuran) (Z = O, R" = Phenyl, R" = H, R"' = iPr): farbloses Öl, Sdp. 100°C/0,01 Torr (Luftbad); $[\alpha]_D^{20}$ = +157° (c = 1,2 in n-Hexan); $^1$H-NMR ($CDCl_3$): δ = 7,27 (s, 5H); 5,08 (t, I = 2,8 Hz, 1H); 4,30 (d, I = 9,8 Hz, 1H); 4,26 (d, I = 7,3 Hz, 1H); 2,8-3,1 (m, 1H); 1,1-2,0 (m, 8H); 1,0 (s, 3H); 0,98/0,72 (2d, I = 7,3 Hz, 6H); 0,93 (s, 6H).

(S)-O-MBF-2-Methyl-1-phenylpropanol [(2S-(2α(R*),3aα,4α,7α,7aα-2,3,3a,4,5,6,7,7a-Octahydro-7,8,8-trimethyl-2-(2-metyl-1-phenylpropoxy)-4,7-methanobenzofuran) (Z = O, R' = H, R" = Phenyl, R"' = iPr): farbloses Öl, Sdp. 100°C/0,01 Torr (Luftbad); $[\alpha]_D^{20}$ = +70° (c = 0,9 in n-Hexan); $^1$H-NMR ($CDCl_3$): δ = 7,25 (s, 5H); 5,40 (t, I = 2,8 Hz, 1H); 4,07 (d, I = 7,4 Hz, 1H); 3,61 (d, I = 9,5 Hz, 1H); 2,7-3,0 (m, 1H); 1,1-2,0 (m, 8H); 0,94/0,72 (2d, I = 7,4 Hz, 6H); 0,82 (s, 6H); 0,58 (s, 3H).

f) Trennung von racemischem 1-(3-Bromphenyl)ethanol mit der MBF-Schutzgruppe:

Acetalisierung: 1,00 g MBP-OH und 1,44 g rac. 1-(3-Bromphenyl)ethanol nach der allgemeinen Vorschrift in Beispiel 3. Trennung: SC des Rohproduktes wie in Beispiel 4a (Eluens: Petrolether/Ether 100:1) ergibt unter gleichzeitiger Abtrennung des überschüssigen 1-(3-Bromphenyl)ethanols 820 mg (45 %) (R)-O-MBP-1-(3-Bromphenyl)ethanol und 610 mg (34 %) (S)-O-MBP-1-(3-Bromphenyl)ethanol.

(R)-O-MBP-1-(3-Bromphenyl)ethanol ([2S-(2α(S*),4aα,5α,8α,8aα)]-3,4,4a,5,6,7,8,8a-Octahydro-8,9,9-trimethyl-2-[1-(3-bromphenylethoxy]-5,8-methano-2H-1-benzopyran) (Z = O, R' = $mBrC_6H_4$, R" = H, R"' = $CH_3$): farbloses Öl. $^1$H-NMR ($CDCl_3$): δ = 7,1-7,5 (m, 4H); 4,63-4,92 (m, 2H); 3,84 (d, I = 10 Hz, 1H); 1,0-2,25 (m, 13 H); 0,93 (s, 3H); 0,90 (s, 6H).

(S)-O-MBP-1-(3-Bromphenyl)ethanol ([2S-(2α(R*),4aα,5α,8α,8aα)-3,4,4a,5,6,7,8,8a-Octahydro-8,9,9-trimethyl-2[1(3-bromphenyl)-ethoxy]-5,8-methano-2H-1-benzopyran) (Z = O, R'= H, R" = $mBrC_6H_4$, R"' = $CH_3$): farblose Kristalle, Schmp. 88-90°C, $^1$H-NMR ($CDCl_3$):δ = 7,1-7,5 (m, 4H); 5,11 (t, I = 7 Hz, 1H); 4,68 (q, I = 6,5 Hz, 1H); 3,45 (d, I = 10 Hz, 1H); 0,80-2,28 (m, 13H); 0,75 (s, 6H); 0,34 (s, 3H).

Abspaltung der Schutzgruppe: durch Methanolyse nach der allgemeinen Arbeitsvorschrift in Beispiel 4b,

g) Trennung von racemischem 1-Tetralol mit der MBP-Schutzgruppe: Acetalisierung: 1,0 g MBP-OH und 1,16 g rac. 1-Tetralol nach der allgemeinen Vorschrift in Beispiel 3. Abtrennung des überschüssigen 1-Tetralols durch SC ergibt 1,54 g (95 %) O-MBP-1-Tetralol (Diastereomerengemisch), farbloses Öl.

Trennung: säulenchromatographisch (Eluens: Petrolether/Ether 80:1) wie in Beispiel 4a) ergibt 0,77 g (48 %) (R)-O-MBP-1-Tetralol und 0,75 g (46 %) (S)-O-MBP-1-Tetralol.

(R)-O-MBP-1-Tetralol ([2S-(2α(S*),4aα,5α,8α,8aα)]-3,4,4a,5,6,7,8,8a-Octahydro-8,9,9-trimethyl-2-[1-(1,2,3,4-tetrahydronaphthyl) oxy]-5,8-methano-2H-1-benzopyran) (Z = O, R' R"' = $C_6H_4$-$(CH_2)_3$, R" = H): farbloses Öl, Sdp. 130°C/0,01 Torr (Luftbad); $[\alpha]_D^{20}$ = +79° (c = 0,54 in Dichlormethan); $^1$H-NMR ($CDCl_3$): δ = 6,9-7,3 (m, 4H); 5,19 (t, I = 7 Hz, 1H); 4,63 (t, I = 6 Hz, 1H), 3,86 (d, I = 9 Hz, 1H); 2,6-2,8 (m, 2H); 1,0-2,3 (m, 14H); 0,90 (s, 9H).

(S)-O-MBP-1-Tetralol-([2S-(2α(R*),4aα,5α, 8α ,8aα)]-3,4,4a,5,6,7,8,8a-Octahydro-8,9,9-trimethyl-2-[1-(1,2,3,4-tetrahydronaphthyl)-oxy]-5,8-methano-2H-1-benzopyran) (Z = O, R' = H, R" R"' = $C_6H_4$-$(CH_2)_3$): farbloses Öl, Sdp. 130°C/0,01 Torr (Luftbad); $[\alpha]_D^{20}$ = +40° (c = 0,54 in Dichlormethan); $^1$H-NMR ($CDCl_3$): δ = 6,9-7,3 (m, 4H); 5,13 (t, I = 7 Hz, 1H); 2,6-2,8 (m, 2H); 1,0-2,3 (m, 14H); 0,99 (s, 3H); 0,92 (s, 6H). Abspaltung der Schutzgruppe durch Methanolyse nach der allgemeinen Arbeitsvorschrift in Beispiel 4b).

(R)-1-Tetralol (II: Z = O, R' R"'= $C_6H_4$-$(CH_2)_3$, R" = H): Aus 70 mg (R)-O-MBP-1-Tetralol werden 24 mg (79 %) (R)-1-Tetralol erhalten, farbloses Öl, $[\alpha]_D^{20}$ = -23° (c = 0,48 in Chloroform).

(S)-1-Tetralol (II: Z = O, R' = H, R"R"' = $C_6H_4$-$(CH_2)_3$): Aus 59 mg (S)-O-MBP-1-Tetralol werden 25 mg (97 %) (S)-1-Tetralol erhalten. Farbloses Öl, $[\alpha]_D^{20}$ = +24° (c = 0,50 in Chloroform).

h) Trennung von racemischem 1,2-Diphenyl-1,2-ethandiol mit der MBF-Schutzgruppe:

Acetalisierung: 1,67 g MBF-OH und 2,20 g rac. 1,2-Diphenyl-1,2-ethandiol nach der allgemeinen Vorschrift in

Beispiel 3. Trennung: SC (Eluens: Petrolether/Ether 10:1) ergibt 0,89 g (27 %) (R)-O-MBF-1,2-Diphenyl-1,2-ethandiol und 1,77 g (53 %) (S)-O-MBF-1,2-Diplienyl-1,2-ethandiol.

(R)-O-MBF-1,2-Diphenyl-1,2-ethandiol ([2R-(2α(R*,R*),3aα,4α,7α,7aα)]-1,2-Diphenyl-1-[(2,3,3a,4,5,6,7,7a-Octahydro-7,8,8-trimethyl-4,7-methanobenzofuran-2-yl)oxy]ethanol) (Z = O, R' = H, R'' = Phenyl, R''' = CHOH-Ph): farblose Kristalle Schmp. 72-74° C (aus Petrolether); $[\alpha]_D^{20}$ = +113,2° (c = 0,92 in Dichlormethan); $^1$H-NMR ($CDCl_3$): δ = 7,14 (s, 10H); 5,45 (t, I = 3 Hz, 1H); 4,72/4,64/4,50/4,42 (dd, 2H); 3,65 (d, I = 9,5 Hz, 1H); 3,29 (s, 1H); 2,6-3,1 (m, 1H); 0,9-1,9 (m, 7H); 0,84 (s, 6H); 0,53 (s, 3H).

(S)-O-MBF-1,2-Diphenyl-1,2-ethandiol-([2R-(2α(S*,S*)]3aα,4α,7α,7aα), 1,2-Diphenyl-1-[(2,3,3a,4,5,6,7,7a-Octahydro-7,8,8-trimethyl-4,7-methanobenzofuran-2-yl)oxy]ethanol) (Z = O, R' = Phenyl, R'' = H, R''' = CHOH-Ph): farbloses Öl, $[\alpha]_D^{20}$ = +104,7° (c = 1,27/Ether); $^1$H-NMR ($CDCl_3$): δ = 7,13 (s, 10H); 5,16 (t, I = 3 Hz, 1H); 4,66 (s, 2H); 3,92 (d, I = 9,5 Hz, 1H); 3,52 (br. s, 1H); 2,6-3,1 (m, 1H); 1,0-1,9 (m, 7H); 0,91/0,87/0,81 (3s, 9H).

**Beispiel 5**: Trennung von racemischen Thiolen

a) Trennung von racemischem 1-Phenylethanthiol mit der MBF-Schutzgruppe:

Acetalisierung: Aus 500 mg MBF-O-MBF und 450 mg rac. 1-Phenylethanthiol nach der allgemeinen Vorschrift in Beispiel 3. Trennung: Das erhaltene Rohprodukt wird durch SC über triethylaminimprägniertes Kieselgel aufgetrennt (Eluens: zuerst Petrolether/Ether 100 : 1, dann 50/1). Ausbeute 311 mg (37 %) (R)-S-MBF-1-Phenylethanthiol, 297 mg (35 %) (S)-S-MBF-1-Phenylethanthiol.

(R)-S-MBF-1-Phenylethanthiol ([2R-(2α(R*),3aα,4α,7α,7aα)]-2,3,3a,4,5,6,7,7a-Octahydro-7,8,8-trimethyl-2-[(1-phenyl-ethyl)thio]-4,7-methanobenzofuran) (Z = S, R' = Phenyl, R'' = H, R''' = $CH_3$): farbloses Öl; $[\alpha]_D^{20}$ = +445° (c = 0,9 in n-Hexan); $^1$H-NMR ($CDCl_3$): δ = 7,2-7,4 (m, 5H); 5,19/5,14/5,11/5,06 (dd, 1H); 4,40 (d, I = 9 Hz, 1H); 4,16 (q, I = 7,5 Hz, 1H); 2,65-3,05 (m, 1H); 1,00-2,25 (m, 10H); 0,95 (s, 3H); 0,88 (s, 6H).

(S)-S-MBF-1-Phenylethanthiol ([2R-(2α(S*),3aα,4α,7α,7aα)]-2,3,3a,4,5,6,7,7a-Octahydro-7,8,8-trimethyl-2-[(1-phenyl-ethyl)thio]-4,7-methanbenzofuran) (Z = S, R' = H, R'' = Phenyl, R''' = $CH_3$): farbloses Öl; $[\alpha]_D^{20}$ = +144° (c = 1,6 in n-Hexan); $^1$H-NMR ($CDCl_3$): δ = 7,2 (m, 5H); 5,68/5,64/5,61/5,57 (dd, 1H); 4,28 (d, I = 9,8 Hz, 1H); 4,15 (q, I = 7 Hz, 1H); 2,6-3,0 (m, 1H); 1,0-2,3 (m, 10H); 0,90/0,87/0,81 (3s, 9H).

Abspaltung der Schutzgruppe: Eine Lösung von 59,5 mg (S)-S-MBF-1-Phenylethanthiol (laut $^1$H-NMR Gehalt von 8 % (R)-Diastereomerem) in 10 ml Methanol wird nach Zugabe einer Spatelspitze p-Toluolsulfonsäure 15 h auf 45° C erwärmt. Es wird mit wäßriger Natriumcarbonatlösung und Petrolether versetzt, die wäßrige Phase mit Petrolether extrahiert und die vereinigten organischen Phasen getrocknet, eingedampft und mit Petrolether chromatographiert: 21,3 mg (82 %) (S)-1-Phenylethanthiol (II: Z = S, R' = H, R'' = Phenyl, R''' = $CH_3$): farbloses Öl; $[\alpha]_D^{20}$ = -52,8° (c = 0,3 in Tetrahydrofuran).

b) Trennung von racemischem 1-Phenylethanthiol mit der MBP-Schutzgruppe:

Acetalisierung: Aus 1,20 g MBP-OH und 0,79 g rac. 1-Phenylethanthiol nach der allgemeinen Vorschrift in Beispiel 3. Trennung: Das erhaltene Rohprodukt wird durch SC über nichtimprägniertes Kieselgel aufgetrennt (Eluens: Petrolether/Ether 100:1). Ausbeute 0,85 g (45 %) (R)-S-MBP-1-Phenylethanthiol, 0,83 g (44 %) (S)-S-MBP-1-Phenylethanthiol.

(R)-S-MBP-1-Phenylethanthiol ([2R-(2α(R*),4aα,5α,8α,8aα)]-3,4,4a,5,6,7,8,8a-Octahydro-8,9,9-trimethyl-2-[(1-phenyl-ethyl)thio]-5,8-methano-2H-1-benzopyran) (Z = S, R' = Phenyl, R'' = H, R''' = $CH_3$): farbloses Öl. Sdp. 120° C/0,01 Torr (Luftbad); $[\alpha]_D^{20}$ = +382° (c = 0,90 in Dichlormethan); $^1$H-NMR ($CDCl_3$): δ = 7,15-7,35 (m, 5H); 4,91 (t, I = 9 Hz, 1H); 4,06 (q, I = 8 Hz, 1H); 3,99 (d, I = 10 Hz; 1H); 1,0-2,2 (m, 13H); 0,95 (s, 3H); 0,92 (s, 6H).

(S)-S-MBP-1-Phenylethanthiol ([2R-(2α(S*),4aα,5α,8α,8aα)]-3,4,4a,5,6,7,8,8a-Octahydro-8,9,9-trimethyl-2-[1-phenyl-ethyl)thio]-5,8-methano-2H-1-benzopyran) (Z = S, R' = H, R'' = Phenyl, R''' = $CH_3$): farbloses Öl, Sdp. 120° C/0,01 Torr (Luftbad); $[\alpha]_D^{20}$ = +129° (c = 0,61 in Dichlormethan); $^1$H-NMR ($CDCl_3$): δ = 7,1-7,3 (m, 5H); 5,37 (t, I = 8 Hz, 1H); 4,09 (q, I = 7 Hz, 1H); 3,93 (d, I = 10 Hz, 1H); 1,0-2,25 (m, 13H); 0,089 (s, 6H); 0,81 (s, 3H).

Abspaltung der Schutzgruppe:

(R)-1-Phenylethanthiol (II: Z = S, R' = Phenyl, R'' = H, R''' = $CH_3$): Eine Lösung von 68 mg (R)-S-MBP-1-Phenylethanthiol in 10 ml Methanol wird nach Zugabe einer Spatelspitze p-Toluolsulfonsäure 10 h unter Rückfluß erhitzt. Dann wird Ether zugesetzt, festes Natriumbicarbonat und Natriumsulfat zugegeben, filtriert, eingedampft und chromatographiert (Petrolether/Ether 100:1). Ausbeute: 20,3 mg (71 %) (R)-1-Phenylethanthiol; $[\alpha]_D^{20}$ = +98° (c = 0,41 in Tetrachlormethan). (S)-1-Phenylethanthiol (II: Z = S, R' = H, R'' = Phenyl, R''' = $CH_3$): Aus 70 mg (S)-S-MBP-1-Phenylethanthiol werden 20,5 mg (70 %) (S)-1-Phenylethanthiol erhalten; $[\alpha]_D^{20}$ = -90° (c = 0,41 in Chloroform).

**Beispiel 6**: Trennung von racemischen Aminen:

Trennung von racemischem 1-Phenylethanamin: Acetalisierung: Eine Lösung von 970 mg MBF-OH und 446 mg rac. 1-Phenylethanamin in 25 ml Petrolether/Ether 1:1 wird nach Zusatz einer Spatelspitze p-Toluolsulfonsäure 12 h bei Raumtemperatur gerührt. Danach wird festes Natriumbicarbonat und Natriumsulfat

zugegeben, filtriert, eingedampft, in abs. Ether aufgenommen, etherische Salzsäure zugesetzt und im Vakuum bei 40° C erneut zur Trockene eingedampft.

Trennung: Der Rückstand wird in möglichst wenig Dichlormethan gelöst, mit der doppelten Menge wasserfreien Tetrahydrofurahs versetzt, im Vakuum bei 40° C auf ein Viertel des Volumens eingeengt und bei -20° C kristallisieren gelassen. Ausb. 490 mg (40 %).

(S)-NBF-1-Phenylethanamin-Hydrochlorid (laut $^1$H-NMR mit 10 % (R)-Diastereomerem verunreinigt). Durch nochmaliges Umkristallisieren aus Dichlormethan/Tetrahydrofuran werden die farblosen Kristalle gereinigt. (2R-(2α(S*),3aβ,4β,7β,7aβ)]-2,3,3a,4,5,6,7,7a-Octahydro-7,8,8-trimethyl-N-(1-phenylethyl)-4,7-methano-2-benzofuranamin, Hydrochlorid

$$(Z = \overset{+}{N}H_2Cl,\ R' = H,\ R'' = Phenyl,\ R''' = CH_3):$$

Schmp. 175-185° C (Zers.), sublimiert unzersetzt bei 150° C/0,01 Torr (Luftbad); $[\alpha]_D^{20}$ = +34,1° (c = 0,9 in Dichlormethan); $^1$H-NMR ($CDCl_3$): δ = 10,0 (br. s, 1H); 9,7 (br. s, 1H); 7,2-7,7 (m, 5H); 5,0 (br. s, 1H); 4,4 (d, I = 9,3 Hz, 1H); 4,2 (br. s, 1H); 2,9-3,4 (m, 1H); 1,0-2,3 (m, 10H); 0,93/0,85/0,76 (3s, 9H).

Die Mutterlauge der Herstellung von (S)-M-MBF-1-Phenylethanamin-Hydrochlorid wird eingedampft, mit Ether digeriert, in wenig Dichlormethan aufgenommen, mit Ether versetzt und kristallisieren gelassen. Es werden 613 mg (50 %) farblose Kristalle erhalten (laut $^1$H-NMR mit 28 % (S)-Diastereomerem verunreinigt). Durch mehrmaliges Umkristallisieren aus Dichlormethan/Ether wird (R)-N-MBF-1-Phenylethanamin-Hydrochlorid rein erhalten. [2R-(2α(R*),3aβ,4β,7β,7aβ)]-2,3,3a,4,5,6,7,7a-Octahydro-7,8,8-trimethyl-N(1-phenylethyl)-4,7-methano-2-benzofuranamin, Hydrochlorid

$$(Z = \overset{+}{N}H_2Cl,\ R' = Phenyl,\ R'' = H,\ R''' = CH_3):$$

farblose Kristalle, Schmp. 212-215° C; $[\alpha]_D^{20}$ = +76,4° (c = 1,6 in Dichlormethan); $^1$H-NMR ($CDCl_3$): δ = 9,76 (br. s, 1H); 7,2-7,7 (m, 5H); 4,63 (d, I = 8,5 Hz, 1H); 4,4 (br. s, 2H); 2,8-3,5 (m, 1H); 1,1-2,6 (m, 10H); 1,02 (s, 3H); 0,89 (s, 6H).

Abspaltung der Schutzgruppe: 317 mg (S)-N-MBF-1-Phenylethanamin in 30 ml Ether werden zehnmal mit je 5 ml 10 proz. wäßriger Zitronensäurelösung versetzt und je 15 min gerührt. Die wäßrigen Phasen werden mit Ether rückgeschüttelt, die vereinigten wäßrigen Phasen mit festem Natriumcarbonat neutralisiert und sorgfältig ausgeethert. Die Etherphase wird mit 10 proz. Essigsäure extrahiert und die saure wäßrige Phase wiederum mit Natriumcarbonat neutralisiert. Es wird ausgeethert, getrocknet und eingedampft. Ausbeute 107 mg (83 %) (S)-1-Phenylethanamin (II: Z = NH, R' = H, R'' = Phenyl, R''' = $CH_3$).

**Beispiel 7**: Diastereomerentrennung von Acetalen aus Cyanhydrinen:

Acetalisierung: Aus 1,0 g MBF-OH und 0,73 g rac. α-Hydroxybenzolacetonitril nach der allgemeinen Vorschrift in Beispiel 3. Reaktionszeit 1 h. Es wird durch Ausschütteln mit wäßriger Natriumbicarbonat- und Natriumbisulfit-Lösung aufgearbeitet. Trennung: 1,55 g (97 %) O-MBF-α-Hydroxybenzolacetonitril (Diastereomerengemisch (R)/(S) ~ 1:1 laut $^1$H-NMR) werden durch SC (Eluens: Petrolether/Ether 20:1) in folgende Fraktionen aufgetrennt: 691 mg (43 %) (R)-O-MBF-α-Hydroxybenzolacetonitril, 309 mg (19 %) Gemisch der Diastereomeren und 483 mg (30 %) (S)-O-MBF-α-Hydroxybenzolacetonitril. (S)-O-MBF-α-Hydroxybenzolacetonitril ([2R-(2α(S*),3aα,4α,7α,7aα)]-α-[(2,3,3a,4,5,6,7,7a-Octahydro-7,8,8-trimethyl-4,7-methanobenzofuran-2-yl)oxy]-benzolacetonitril) (Z = O, R' = Phenyl, R'' = H, R''' = CN): farblose Kristalle, Schmp. 83-86° C (aus Petrolether); $^1$H-NMR ($CDCl_3$): δ = 7,3-7,6 (s, 5H); 5,3-5,45 (m, 2H); 4,51 (d, I = 9 Hz, 1H); 2,8-3,3 (m, 1H); 1,0-2,3 (m, 7H); 1,00 (s, 3H); 0,92 (s, 6H).

(R)-O-MBF-α-Hydroxybenzolacetonitril ([2R-(2α(R*),3aα,4α,7α,7aα)]-α-[(2,3,3a,4,5,6,7,7a-Octahydro-7,8,8-trimethyl-4,7-methanobenzofuran-2-yl)oxy]benzolacetonitril) (Z = O, R' = H, R'' = Phenyl, R''' = CN): farblose Kristalle, Schmp. 67-70° C (aus Petrolether); $[\alpha]_D^{20}$ = +120° (c = 7,0 in THF).

$^1$H-NMR ($CDCl_3$): δ = 7,3-7,6 (s, 5H); 5,71 (d, I = 4,6 Hz, 1H); 5,51 (s, 1H); 4,23 (d, I = 9,3 Hz, 1H); 2,65-3,1 (m, 1H); 1,05-2,2 (m, 7H); 0,92 (s, 6H); 0,88 (s, 3H),

**Beispiel 8**:

Herstellung optisch aktiver Imidoesterhydrochloride durch Methanolyse MBF-geschützter Cyanhydrine sowie Verseifung zu optisch aktiven Estern:

(S)-α-Hydroxy-benzolethanimidsäuremethylester-Hydrochlorid

$(II: Z = O, R' = Phenyl, R'' = H, R''' = C(OCH_3)\overset{+}{N}H_2Cl^-)$:

Eine Lösung von 204 mg (S)-O-MBF-α-Hydroxybenzolacetonitril in 3 ml wasserfreiem Ether wird mit 2 ml etherischer Salzsäure versetzt und 17 h bei 5°C stehengelassen. Der ausgefallene Niederschlag wird abgenutscht und im Vakuum bei 40°C getrocknet. Ausbeute 23,2 mg (93 %) farblose, hygroskopische Kristalle, Schmp. 84-87°C (Zers.). Durch Verdunstenlassen der Mutterlauge und Umkristallisieren aus Petrolether werden 101 mg (80 %) MBF-O-MBF zurückgewonnen.

(S)-α-Hydroxybenzolessigsäuremethylester ((S)-Mandelsäuremethylester) (II: Z = O, R' = Phenyl, R'' = H, R''' = $COOCH_3$): Eine Lösung von 105 mg (S)-α-Hydroxybenzolethanimidsäuremethylester-Hydrochlorid in 10 ml 0,2 N HCl wird mit Ether überschichtet und 3 h gerührt. Die Etherphase wird abgetrennt, die wäßrige Phase noch zweimal mit Ether extrahiert, die vereinigten organischen Phasen mit Natriumbicarbonat-Lösung gewaschen, getrockret und eingedampft. Es werden 75,1 mg (87 %) farblose Kristalle erhalten, Schmp. 54-55°C; $[\alpha]_D^{20} = +168°$ (c = 1,36 in Benzol).

(R)-α-Hydroxy-benzolethanimidsäuremethylester-Hydrochlorid

$(II: Z = O, R' = H, R'' = Phenyl, R''' = C(OCH_3)\overset{+}{N}H_2Cl^-)$:

Aus 199 mg (R)-O-MBF-α-Hydroxybenzolacetonitril werden nach der für das (S)-Diastereomere angegebenen Vorschrift 96,9 mg (75 %) farblose, hygroskopische Kristalle erhalten, Schmp. 84-87°C (Zers.).

(R)-α-Hydroxy-benzolessigsäuremethylester ((R)-Mandelsäuremethylester) (II: Z = O, R' = H, R'' = Phenyl, R''' = $COOCH_3$): aus 61 mg (R)-α-Hydroxybenzolethanimidsäuremethylester-Hydrochlorid, wie für das (S)-Diastereomere angegeben. Ausbeute 41,7 mg (84 %) farblose Kristalle, Schmp. 54-55°C; $[\alpha]_D^{20} = -176°$ (c = 0,83 in Benzol).

**Beispiel 9**:

Enantiomerenanreicherung durch enantioselektive Acetalisierung

a) 1-Phenylethanol aus Diastereomerengemisch der enantioselektiven Acetalisierung mit 4 mol rac. 1-Phenylethanol: Aus 421 mg O-MBF-1-Phenylethanol (Diastereomerengemisch (R)/(S)~ 2-1 laut $^1$H-NMR, hergestellt nach dem Verfahren in Beispiel 4a)) werden nach der Abspaltung der Schutzgruppe durch Methanolyse nach der Arbeitsvorschrift in Beispiel 4a) (SC: Eluens: Petrolether/Ether 20:1, dann Ether) 149 mg (87 %) 1-Phenylethanol als farbloses Öl erhalten; $[\alpha]_D^{20} = +20,3°$ (c = 2,1 in Chloroform).

b) 1,2-Diphenyl-2-hydroxyethanon aus Diastereomerengemisch der enantioselektiven Acetalisierung mit 3,9 mol rac. 1,2-Diphenyl-2-hydroxyethanon (Diastereomerengemisch (R)/(S) ~ 3:7 laut $^1$H-NMR). Acetalisierung: 2,078 g MBF-O-MBF und 9,145 g rac. 1,2-Diphenyl-2-hydroxyethanon nach der allgemeinen Vorschrift in Beispiel 3. Trennung: Zunächst wird O-MBF-1,2-Diphenyl-2-hydroxyethanon von überschüssigem 1,2-Diphenyl-2-hydroxyethanon durch Digerieren mit wenig Dichlormethan abgetrennt, dann SC (Eluens: Petrolettier/Ether 30:1).

Methanolyse: O-MBF-1,2-Diphenyl-2-hydroxyethanon wird mit 6 ml Methanol und 10 mg p-Toluolsulfonsäure versetzt, die Kristalle abgesaugt und mit Petrolether gewaschen: 1,83 g (78 %) 1,2-Diphenyl-2-hydroxyethanon; $[\alpha]_D^{26} = +45,0°$ (c = 0,52 in Chloroform).

ASYMMETRISCHE INDUKTION: Folgendes Reaktionsschema erläutert die in den beiden folgenden Beispielen angeführten

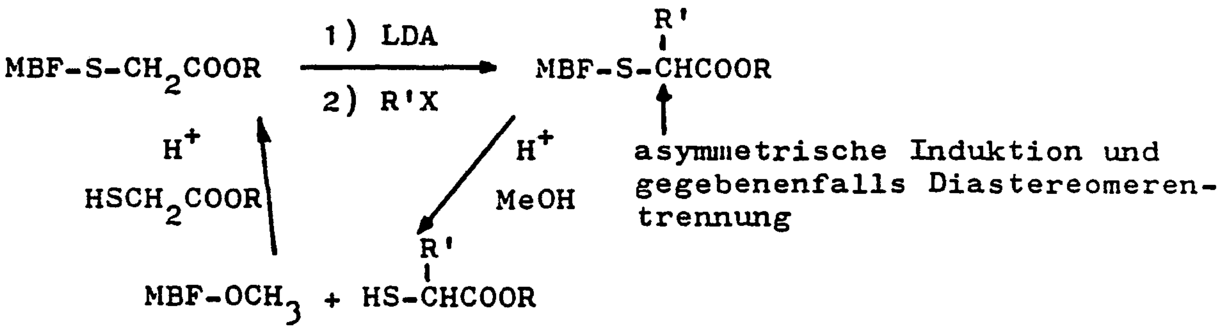


I II

**Beispiel 10**: Asymmetrische Methylierung von Mercaptoessigssäure:

Acetalisierung: S-MBF-Mercaptoessigsäure ([2R-(2α,3aα,4α,7α,7aα)]-[(2,3,3a,4,5,6,7,7a-Octahydro-7,8,8-trimethyl-4,7-methanobenzofuran-2-yl)thio]-essigsäure) (Z = S, R' = R'' = H, R''' = COOH): Eine Lösung von 376 mg O-MBF-Methanol und 214 mg Mercaptoessigsäure in 5 ml Dichlormethan wird mit 3 Tropfen Chlorwasserstoff-gesättigtem Ether versetzt. Nach 3 h wird das Reaktionsgemisch zwischen Ether und 2 N KOH verteilt und die organische Phase mehrmals mit 2 N KOH extrahiert. Unter Eiskühlung werden die alkalischen Phasen mit halbkonz. Salzsäure angesäuert und mit Dichlormethan extrahiert. Die organische Phase wird mit verdünter Salzsäure gewaschen, getrocknet, eingedampft und überschüssige Säure im Feinvakuum bei 50°C abdestilliert. Ausbeute 430 mg (90 %) farbloses Öl; Sdp. 120°C/0,001 Torr (Luftbad); $^1$H-NMR ($CDCl_3$): δ = 5,80/5,78/5,72/5,70 (dd, 1H); 4,36 (d, I = 9,5 Hz, 1H); 2,7-3,75 (m, 3H); 1,0-2,4 (m, 7H); 0,97/0,90/0,84 (3s, 9H).

Acetalisierung auch aus MBF-OH bzw. MBF-O-MBF mit Chlorwasserstoff-gesättigtem Ether als Katalysator nach der allgemeinen Vorschrift in Beispiel 3, wobei überschüssige Mercaptoessigsäure im Feinvakuum bei 50°C abdestilliert wird.

Methylierung: In eine Lösung von 2,45 ml Diisopropylamin in 10 ml wasserfreiem tetrahydrofuran werden unter $N_2$ bei -30°C 7,8 ml einer 1,6 N Lösung von n-Butyllithium in n-Hexan eingespritzt. Nach 45 min wird auf -75°C abgekühlt und 1,12 g S-MBF-Mercaptoessigsäure in 5 ml wasserfreiem Tetrahydrofuran zugetropft. Nach 45 min bei -75°C werden +400 µl Jodmethan zugetropft. Es wird 1 h bei -75°C, dann 1 h bei -60°C gehalten und anschließend innerhalb von 2 h auf -10°C erwärmen gelassen. Es wird Ether und verdünnte Natronlauge zugegeben. Die organische Phase wird mit Natronlauge mehrmals extrahiert, die vereinigten alkalischen Phasen mit Petrolether gewaschen und unter Eiskühlung mit halbkonzentrierter Salzsäure angesäuert. Es wird mit Dichlormethan extrahiert, mit wenig verd. Salzsäure gewaschen, getrocknet und eingedampft. Ausbeute 1,05 g (89 %) (R)-S-MBF-2-Mercaptopropansäure ([2R-(2α(R*),3aα,4α,7α,7aα)]-2-[(Octahydro-7,8,8-trimethyl-4,7-methanobenzofuran-2-yl)-thio]propansäure) (Z = S, R' = H, R'' = $CH_3$, R''' = COOH): farbloses Öl, Sdp. 145°/0,001 Torr (Luftbad); $^1$H-NMR ($CDCl_3$): δ = 9,7 (s, 1H); 5,90/5,86/5,79 (dd, 1H); 4,4 (d, I = 9,5 Hz, 1H); 3,65 (q, I = 7 Hz, 1H); 2,7-3,1 (m, 1H); 1,1-2,4 (m, 10H); 0,99/0,92/0,88 (3s, 9H). Folgende abweichende Lagen werden dem (S)-Diastereomeren zugeordnet: 5,83/5,79/5,75/5,71 (dd); 4,44 (d, I = 9,5 Hz); 3,60 (q, I = 7 Hz); 0,85 (s). Aus dem Integral wird ein Gehalt von ~ 23 % (S)-Diastereomerem errechnet.

Abspaltung der Schutzgruppe unter gleichzeitiger Veresterung: 260 mg S-MBF-2-Mercaptopropansäure ((R)/(S) ~ 3 : 1) werden in 30 ml Dichlormethan und 20 ml Methanol gelöst und nach Zusatz von 0,1 ml konz. Schwefelsäure unter $N_2$ 17 h unter Rückfluß erhitzt. Es wird mit gesättigter Natriumbicarbonatlösung neutralisiert, die organische Phase abgetrennt und die wäßrige Phase mit Ether extrahiert. Die vereinigten organischen Phasen werden getrocknet und das Lösungsmittel bei schwachem Vakuum abdestilliert. Nach Destillation bei 80°C/30 Torr (Luftbad) werden 100 mg (91 %) 2-Mercaptopropansäuremethylester erhalten. (Im Rückstand verbleiben 190 mg (99 %) O-MBF-Methanol.) Farbloses Öl; Drehwert der Säure (nach Versein fung): $[\alpha]_D^{20}$: = +26,8° (c = 1,2 in Chloroform).

**Beispiel 11**:

Asymmetrische Induktion: Alkylierung mit anschließender Diastereomerentrennung

a) Diastereomerenanreicherung von S-MBF-2-Mercaptopropansäure aus der asymmetrischen Methylierung (siehe Bsp. 10) nach Veresterung. Veresterung: Eine Lösung von 579 mg Rohprodukt aus Methylierung von S-MBF-Mercaptoessigsäure in 20 ml Ether wird portionsweise bis zur bleibenden Gelbfärbung und Aufhören der $N_2$-Entwicklung mit etherischer Diazomethanlösung versetzt. Anschließend wird mit wäßriger Natriumcarbonatlösung und 0,5 N HCl gewaschen, getrocknet und eingedampft. Trennung: Das erhaltene Rohprodukt (560 mg; 94 %) wird über triethylaminimprägniertes Kieselgel säulenchromatographischer. Folgende Produktfraktionen werden erhalten: 170 mg Diastereomerengemisch ((R)/(S) = 40:60), 114 mg Diastereomerengemisch ((R)/(S) = 78/22) und 270 mg reiner (R)-S-MBF-2-Mercaptopropansäuremethylester ([2R-(2α(R*),3aα,4α,7α,7aα)]-2-[(2,3,3a,4,5,6,7,7a-Octahydro-7,8,8-trimethyl-4,7-methanobenzofuran-2-yl) thio]-propansäuremethylester) (Z = S, R' = H, R'' = $CH_3$, R''' = $COOCH_3$): farbloses Öl, Sdp. 110°C/0,01 Torr (Luftbad) $[\alpha]_D^{20}$ +373° (c = 1,26 in n-Hexan); $^1$H-NMR ($CDCl_3$): δ = 5,83/5,80/5,75/5,72 (dd, 1H); 4,36 (d, I = 9,5 Hz, 1H); 3,74 (s, 3H); 3,63 (q, I = 7 Hz, 1H); 2,7-3,2 (m, 1H); 1,1-2,5 (m, 10H); 0,98/0,92/0,89 (3s, 9H).

b) Asymmetrische Ethylierung mit anschließender Diastereomerentrennung: In eine Lösung von 2,23 ml Diisopropylamin in 10 ml wasserfreiem Tetrahydrofuran werden unter $N_2$ bei -30°C 7,12 ml einer 1,6 N Lösung von n-Butyllithium in n-Hexan eingespritzt. Nach 30 min werden 2 ml wasserfreies HMPA zugegeben. Es wird auf -75°C gekühlt und 1,03 g S-MBF-Mercaptoessigsäure in 5 ml wasserfreiem Tetrahydrofuran zugetropft. Nach 45 min bei -75°C wird 1 ml Jodethan zugetropft und das Reaktionsgemisch 1 h bei -35°C und anschließend 4 h bei -15°C gehalten. Aufarbeitung nach dem Verfahren in Beispiel 10; Ausbeute 1,11 g (97 %) (R)-S-MBF-2-Mercaptobutansäure ([2R-(2α(R*),3aα,4α,7α,7aα)]-2-[(Octahydro-7,8,8-trimethyl-4,7-methanobenzofuran-2-yl) thio]-butansäure) (Z = S, R' = H, R'' = Et, R''' = COOH): farbloses Öl, Sdp. 145°C/0,03 Torr (Luftbad); $^1$H-NMR ($CDCl_3$): δ = 5,78/5,75/5,71/5,68 (dd, 1H); 4,33 (d, I = 9,5 Hz, 1H); 3,39 (t, 1H); 2,7-3,2 (m, 1H); 1,1-2,4 (m, 9H); 1,02 (t, I = 7 Hz, 3H); 0,97/0,91/0,87 (3s, 9H). Folgende abweichende Lagen werden dem (S)-Diastereomeren zugeordnet: 3,36 (t); 0,84 (s). Die Integration ermöglicht eine Abschätzung des Gehaltes an (S)-Diastereomerem mit 20 %.

Veresterung: 660 mg Rohprodukt aus Ethylierung von S-MBF-Mercaptoessigsäure werden nach der Vorschrift in Bsp. 11a) umgesetzt. Trennung: Das erhaltene Rohprodukt (620 mg; 90 %) wird durch SC über triethylaminimprägniertes Kieselgel aufgetrennt (Eluens: Petrolether/Ether 20:1). Folgende Produktfraktionen wurden erhalten: 162 mg Diastereomerengemisch und 315 mg (R)-S-MBF-2-Mercaptobutansäuremethylester (Gehalt an (S)-Diastereomerem ⩽ 3 %). [2R-(2α(R*),3aα,4α,7α,7aα)]-2-[(2,3,3a,4,5,6,7,7a-Octahydro-7,8,8,-trimethyl-4,7-methanobenzofuran-2-yl)thio]-butansäuremethylester) (Z = S, R' = H, R'' = Et, R''' = $COOCH_3$): farbloses Öl, Sdp. 115°/0,03 Torr (Luftbad); $[\alpha]_D^{20}$ = +356° (c = 2,9 in n-Hexan); $^1$H-NMR ($CDCl_3$): δ = 5,76/5,73/5,68/5,65 (dd, 1H); 4,33 (d, I = 9,5 Hz, 1H); 3,73 (s, 3H); 3,40 (t, I = 7 Hz, 1H); 2,7-3,2 (m, 1H); 1,1-2,35 (m, 9H); 0,98 (t, 3H); 0,98/0,92/0,87 (3s, 9H).

Abspaltung der Schutzgruppe unter gleichzeitiger Veresterung: Aus 168 mg (R)-S-MBF-2-Mercaptobutansäuremethylester nach der in Bsp. 10 angegebenen Vorschrift. Nach Destillation bei 100°C/15 Torr (Luftbad) werden 61 mg (81 %) (R)-2-Mercapto-butansäuremethylester (II: Z = S, R' = COOCH , R'' = H, R''' = Et) erhalten: farbloses Öl; Drehwert der freien Säure (nach Verseifung); $[\alpha]_D^{20}$ = +29° (c = 0,14 in Ether).

c) Asymmetrische Methylierung von Mercaptoessigsäuremethylester und Diastereomerentrennung:

Acetalisierung: Aus 3,6 g MBF-OH und 2,14 g Mercaptoessigsäuremethylester nach der allgemeinen Vorschrift in Bsp. 3) mit 6 Tropfen Chlorwasserstoff-gesättigtem Ether zur Katalyse. SC Elumens: Petrolether/Ether 10:1). Es werden 4,7 g (90 %) farbloses Öl erhalten: S-MBF-Mercaptoessigsäuremethylester ([2R-(2α,3aα,4α,7α,7aα)]-2,3,3a,4,5,6,7,7a-Octahydro-7,8,8-trimethyl-4,7-methanobenzofuran-2-yl)thio]essigsäuremethylester) (Z = S, R' = R'' = H, R''' = $COOCH_3$): Sdp. 105°C/0,001 Torr (Luftbad); $^1$H-NMR ($CDCl_3$): δ = 5,77/5,74/5,70/5,67 (dd, 1H); 4,33 (d, I = 9,5 Hz, 1H); 3,74 (s, 3H); 2,7-3,03 (m, 1H); AB-Signal ($\delta_A$ = 3,20, $\delta_B$ = 3,36, I = 13,8 Hz, 2H); 1,0-2,4 (m, 7H); 0,97/0,90/0,84 (3s, 9H).

Methylierung: In eine Lösung von 1,53 ml Diisopropylamin in 5 ml wasserfreiem Tetrahydrofuran werden unter $N_2$ bei -50°C 4,9 ml einer 1,6 N Lösung von n-Butyllithium in n-Hexan eingespritzt. Nach 20 min werden 3 ml HMPA zugegeben und nach weiteren 10 min auf -75°C abgekühlt und 1,01 g S-MBF-Mercaptoessigsäuremethylester in 5 ml wasserfreiem Tetrahydrofuran zugetropft. Nach 40 min bei -80°C werden 0,36 ml Jodmethan zugetropft. Die Temperatur wird 2 h bei -80°C gehalten, anschließend auf -40°C erwärmen gelassen und nach 30 min Ether und wäßrige Natriumbicarbonatlösung zugegeben. Die wäßrige Phase wird ausgeethert, die vereinigten organischen Phasen mit 0,1 N HCl und Wasser gewaschen, getrocknet unnd eingedampft. Ausbeute 1,0 g (92 %) farbloses Öl.

Trennung: Bei der anschließenden Säulenchromatographie analog zum identischen Produkt aus Bsp. 11a) werden 0,55 g (51 %) (R)-S-MBF-2-Mercaptopropansäuremethylester isoliert.

## Patentansprüche

1. Chirale enantiomerenreine Verbindungen der allgemeinen Formel

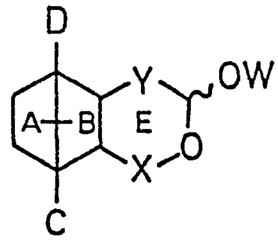

(I),

worin an ein Bicyclo[2.2.1]heptan-Ringsystem, wobei A, B, C, D die Bedeutung H oder Methyl in beliebiger Kombination haben, vorzugsweise ein Bornan-Ringsystem, ein 5- oder 6-gliedriger Lactolring (E) stereospezifisch anelliert ist, worin X und Y jeweils einen Rest $(CR^1R^2)_n$ mit n = 0 bis 2 und $R^1$ und $R^2$ Wasserstoff, Niederalkyl oder Aryl in beliebiger Kombination bedeuten, soweit die anomere Selektivität bei Acetalisierungsreaktionen nicht beeinträchtigt wird, und W Wasserstoff, substituiertes oder unsubstituiertes Alkyl, Cycloalkyl, oder das Ringsystem selbst, insbesondere H, das Ringsystem selbst oder Niederalkyl, bedeutet bzw. für den Fall, als W Wasserstoff ist, deren Anhydroverbindungen der allgemeinen Formel

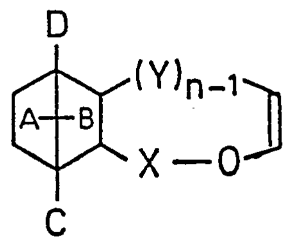

(V),

worin A, B, C, D, X und Y die gleiche Bedeutung wie in der hydratisierten Form haben und n 1 oder 2 bedeutet.

2. Chirale enantiomerenreine Verbindungen ((R)-Enantiomer- bzw. (S)-Enantiomer) der allgemeinen Formel

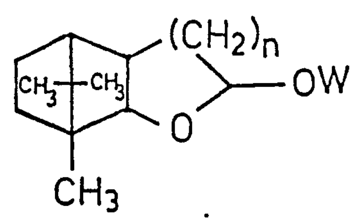

(I a),

worin W und n die in Anspruch 1 angegebene Bedeutung hat und n 1 oder 2 bedeutet, bzw. für den Fall als W Wasserstoff ist, deren Anhydroverbindungen der allgemeinen Formel

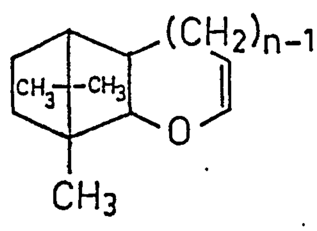

(Va),

worin n 1 oder 2 bedeutet.

3. Chirale, enantiomerenreine (R)-Enantiomere und (S)-Enantiomere der allgemeinen Formel

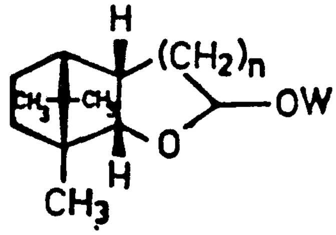

(Ib),

worin W die in Anspruch 1 angegebene Bedeutung hat und n 1 oder 2 bedeutet, bzw. für den Fall als W Wasserstoff ist deren Anhydroverbindungen der allgemeinen Formel

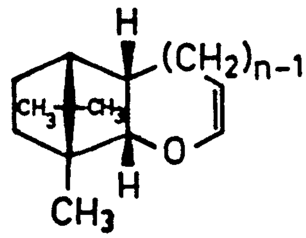

(Vb),

worin n die obige Bedeutung hat.

4. (2α,3aα,4α,7α,7aα)-2,3,3a,4,5,6,7,7a-Octahydro-7,8,8-trimethyl-4,7-methanobenzofuran-2-ol (R)-Enantiomer und (S)-Enantiomer (Fig.1).

5. (2α(2'R*,3'aS*,4R*,7'R*,7'aS*),3aα,4α,7α,7aα)-2,2'-Oxy-bis-[2,3,3a,4,5,6,7,7a-Octahydro-7,8,8-trimethyl-4,7-methanobenzofuran] (R)-Enantiomer und (S)-Enantiomer (Fig.2).

6. (2α,3aα,4α,7α,7aα)-2,3,3a,4,5,6,7,7a-Octahydro-2-methoxy-7,8,8-trimethyl-4,7-methanobenzofuran (R)-Enantiomer und (S)-Enantiomer (Fig.3).

7. (2α,3aα,4α,7α,7aα)-2,3,3a,4,5,6,7,7a-Octahydro-2-ethoxy-7,8,8-trimethyl-4,7-methanobenzofuran (R)-Enantiomer und (S)-Enantiomer (Fig.4).

8. (2α,3aα4α,7α,7aα)-2,3,3a,4,5,6,7,7a-Octahydro-2-isopropoxy-7,8,8-trimethyl-4,7-methanobenzofuran (R)Enantiomer und (S)-Enantiomer (Fig.5).

9. (2α,4aα,5α,8α,8aα)-3,4,4a,5,6,7,8,8a-Octahydro-8,9,9-trimethyl-2H-5,8-methano-1-benzopyran-2-ol (R)-Enantiomer und (S)-Enantiomer (Fig.6).

10. (2α(2'R*,4'aS*,5'R*,8'R*,8'aS*),4aα,5α,8α,8aα)-2,2'-Oxybis-[3,4,4a,5,6,7,8,8a-Octahydro-8,9,9-trimethyl-2H-5,8-methano-1-benzopyran] (R)-Enantiomer und (S)-Enantiomer (Fig.7).

11. (2α,4aα,5α,8α,8aα)-3,4,4a,5,6,7,8,8a-Octahydro-2-methoxy-8,9,9-trimethyl-2H-5,8-methano-1-benzopyran (R)-Enantiomer und (S)-Enantiomer (Fig.8).

12. (3aα,4α,7α,7aα)-3a,4,5,6,7,7a-Hexahydro-7,8,8-trimethyl-4,7-metha-nobenzofuran (R)-Enantiomer und (S)-Enantiomer (Fig.9).

13. (4aα,5α,8α,8aα)-4a,5,6,7,8,8a-Hexahydro-8,9,9-trimethyl-4-H-5,8-methanobenzopyran (R)-Enantiomer und (S)-Enantiomer (Fig.10).

14. Verfahren zur Herstellung von chiralen, enantiomerenreinen Verbindungen der allgemeinen Formel

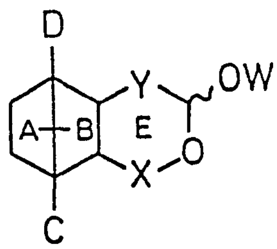

(I),

worin an ein Bicyclo[2.2.1]heptan-Ringsystem, in welchem A, B, C, D die Bedeutung H oder Methyl in beliebiger Kombination haben, vorzugsweise ein Bornan-Ringsystem, ein 5- oder 6-gliedriger Lactolring (E) stereospezifisch anelliert ist, worin X und Y jeweils einen Rest $(CR^1R^2)_n$ mit $n = 0$ bis 2 und $R^1$ und $R^2$ Wasserstoff, Niederalkyl oder Aryl in beliebiger Kombination bedeuten, soweit die anomere Selektivität bei Acetalisierungsreaktionen nicht beeinträchtigt wird, und W Wasserstoff substituiertes oder unsubstituiertes Alkyl, Cycloalkyl oder das Ringsystem selbst, insbesonders H, das Ringsystem selbst oder Niederalkyl bedeutet, bzw. für den Fall, als W Wasserstoff ist, deren Anhydroverbindungen der allgemeinen Formel

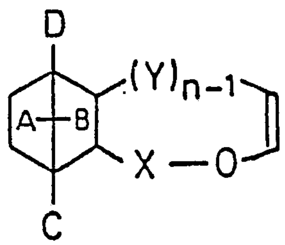

(V),

worin A, B, C, D, X und Y die gleiche Bedeutung wie in der hydratisierten Form haben und n 1 oder 2 bedeutet dadurch gekennzeichnet, daß ein Lacton der allgemeinen Formel

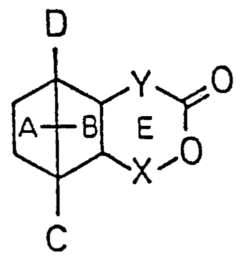

(VI),

worin A, B, C, D, X und Y obige Bedeutung haben, zum Lactol der allgemeinen Formel I (W = H) reduziert wird und, gegebenenfalls dieses durch Säurekatalyse in inertem Lösungsmittel in seine selbstkondensierte Form (W = Ringsystem selbst) übergeführt wird

oder gegebenenfalls dieses durch Behandeln mit katalytischen Mengen einer starken Säure in Gegenwart eines acyclischen oder cyclischen Alkohols , vorzugsweise eines niederen Alkohols, in jene Verbindungen der allgemeinen Formel I, in denen W substituiertes oder unsubstituiertes Alkyl oder Cycloalkyl, vorzugsweise Niederalkyl bedeuten, übergeführt wird

oder gegebenenfalls dieses durch Behandeln mit wasserentziehenden Mitteln, vorzugsweise anorganisches Säurechlorid/Base, zum Enolether der allgemeinen Formel V dehydratisiert wird.

15. Verfahren nach Anspruch 14, dadurch gekennzeichnet, daß eine chirale, enantiomerenreine Verbindung ((R)-Enantiomer- bzw. (S)-Enantiomer) der allgemeinen Formel

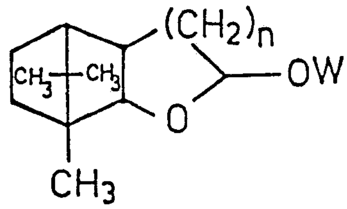

(Ia),

worin W die in Anspruch 14 angegebene Bedeutung hat und n 1 oder 2,bedeutet, und in welcher der Lactolring an ein Bornangerüst anelliert ist, hergestellt wird.

16. Verwendung von chiralen, enantiomerenreine Verbindungen der allgemeinen Formeln I bzw. V, vorzugsweise von Verbindungen der allgemeinen Formeln Ia bzw. Va sowie Ib bzw. Vb, gemäß den Ansprüchen 1 bis 3 und insbesondere den Verbindungen gemäß den Ansprüchen 4 bis 13 zum Schutz funktioneller -OH, -SH, -NH-Gruppen, indem eine Verbindung der allgemeinen Formel I oder Ia oder Ib bzw. V oder Va oder Vb oder eine Verbindung gemäß den Ansprüchen 4 bis 13 unter Säurekatalyse mit einer Verbindung der allgemeinen Formel

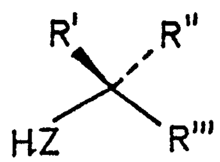

(II),

umgesetzt werden, worin Z = O, S oder NH ist und die Reste R′, R″ und R‴ Wasserstoff und voneinander unabhängige oder miteinander verknüpfte substituierte oder unsubstituierte Alkyl, Aryl, Heteroaryl, Carbonyl, Carboxyl oder Nitrilgruppen in beliebigen Kombinationen bedeuten, sofern diese die Reaktivität von Verbindungen der allgemeinen Formel II mit Verbindungen der allgemeinen Formel I bzw. V nicht beschränken,

wobei in Acylverbindungen CR'R" auch für

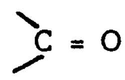 steht ,

insbesondere Wasserstoff, Alkyl, 1-Hydroxyalkyl, 1-Oxoalkyl, Aryl, substituiertes Aryl, Heteroaryl, Aroyl, Heteroaroyl, Carboxyl, Alkoxycarbonyl, Aryloxycarbonyl, Aminocarbonyl und Nitril, wobei für cyclische Strukturen R' und R" zusammen vorzugsweise $-(CR^1R^2)_n-$, worin n = 3 bis 7 ist und $R^1$ und $R^2$ Alkyl und Aryl bedeuten, sowie $-O-Aryl-(CR^1R^2)_n-$, worin n = 2 bis 4 und $R^1$ und $R^2$ Alkyl oder Aryl in beliebiger Kombination darstellen.

17. Verwendung von chiralen enantiomerenreine Verbindungen der allgemeinen Formeln I bzw. V, vorzugsweise von Verbindungen der allgemeinen Formeln Ia bzw. Va sowie Ib bzw. Vb gemäß den Ansprüchen 1 bis 3 und insbesondere den Verbindungen gemäß den Ansprüchen 4 bis 13 zur Racemattrennung, indem eine Verbindung der allgemeinen Formel I oder Ia oder Ib bzw. V oder Va oder Vb oder eine Verbindung gemäß den Ansprüchen 4 bis 13 mit einer racemischen Verbindung der allgemeinen Formel II gemäß Anspruch 16, welche eine -OH, -SH, oder -NH-Funktion möglichst in Nachbarschaft zu einem chiralen Zentrum aufweist, unter Säurekatalyse zu einem Gemisch der Diastereomeren der allgemeinen Formel

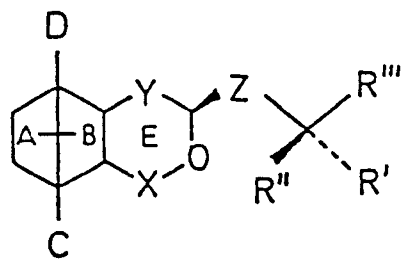 (III) ,

umgesetzt wird, worin A, B, C, D, E, X und Y die in Anspruch 1 angegebene Bedeutung und Z, R', R" und R"' die in Anspruch 16 angegebene Bedeutung haben und diese Diastereomeren, vorzugsweise durch Chromatographie oder Kristallisation, getrennt werden, worauf die Schutzgruppen wieder unter Säurekatalyse, vorzugsweise Methanolyse oder Hydrolyse, abgespalten wird.

18. Verwendung von chiralen, enantiomerenreine Verbindungen der allgemeinen Formeln I bzw. V, vorzugsweise von Verbindungen der allgemeinen Formeln Ia bzw. Va sowie Ib bzw. Vb gemäß den Ansprüchen 1 bis 3 und insbesondere den Verbindungen gemäß den Ansprüchen 4 bis 13, zur Herstellung optisch aktiver Imidoesterhydrochloride sowie optisch aktiver Ester, indem eine Verbindung der allgemeinen Formel I oder Ia oder Ib bzw. V oder Va oder Vb oder eine Verbindung gemäß den Ansprüchen 4 bis 13 mit einem Cyanhydrin der allgemeinen Formel II (Z = O, R' = CN) gemäß Anspruch 16, zum Gemisch der Diastereomeren der allgemeinen Formel III umgesetzt wird, worauf nach Diastereomerentrennung unter Zusatz eines geringen Überschusses an Alkohol und unter Säurekatalyse die Imidoesterhydrochloride erhalten werden, welche gegebenenfalls zu den optisch aktiven Estern verseift werden.

19. Verwendung von chiralen, enantiomerenreine Verbindungen der allgemeinen Formeln I bzw. V, vorzugsweise von Verbindungen der allgemeinen Formeln Ia bzw. Va sowie Ib bzw. Vb, gemäß den Ansprüchen 1 bis 3 und insbesondere den Verbindungen gemäß den Ansprüchen 4 bis 13 zur Gewinnung optisch angereicherter Alkohole durch enantioselektive Acetalisierung, indem ein racemischer Alkohol der allgemeinen Formel II, worin vorzugsweise Z = O, R' = H und R" oder R"' = Aryl ist, mit einer Verbindung der allgemeinen Formel I oder Ia oder Ib bzw. V oder Va oder Vb oder einer Verbindung gemäß den Ansprüchen 4 bis 13 selektiv acetalisiert und anschließend von der Schutzgruppe abgespalten wird.

20. Verwendung von chiralen, enantiomerenreine Verbindungen der allgemeinen Formeln I bzw. V, vorzugsweise von Verbindungen der allgemeinen Formeln Ia bzw. Va, sowie Ib bzw. Vb, gemäß den Ansprüchen 1 bis 3 und insbesondere den Verbindungen gemäß den Ansprüchen 4 bis 13 zur Herstellung optisch aktiver Carbonyl-, Carboxyl- oder Hydroxylverbindungen, indem eine Verbindung der allgemeinen Formel I oder Ia oder Ib bzw. V oder Va oder Vb oder eine Verbindung gemäß den Ansprüchen 4 bis 13 mit einer prochiralen Verbindung der allgemeinen Formel II, welche eine Carbonyl-, Carboxyl- oder Nitril-Funktion trägt, umgesetzt, in den prochiralen Rest der erhaltenen Verbindungen der allgemeinen Formel III durch eine stereospezifische Reaktion Asymmetrie induziert wird und später die Schutzgruppe, gegebenenfalls nach einem Diastereomerentrennungsschritt abgespalten wird, wobei der nicht chirale Rest-ZCR'R"R"' vorzugsweise $SCH_2COOR$ (R = H, Alkyl, Aryl) bedeutet und die asymmetrische Induktion vorzugsweise durch C-Alkylierung nach Deprotonierung erfolgt.

## Claims

1. Chiral, enantiomerically pure compounds of the general formula

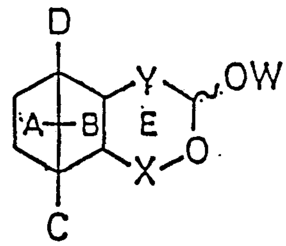

(I)

wherein a 5 or 6 membered lactol ring (E) with X or Y meaning $(CR^1R^2)_n$, with n = 0 to 2 and $R^1$ and $R^2$ = H, lower alkyl or aryl in any combination, which does not impair the anomeric selectivity of the compounds in forming acetals, is fused in stereospecific manner to a bicyclo [2.2.1] heptan system with A, B, C, D, representing H or methyl in any combination, preferably a bornane ring dystem, and wherein W represents hydrogen, substituted or unsubstituted alkyl, cycloalkyl or the ring system itself preferably H, the ring system itself or loweralkyl, respectively in case of W representing hydrogen the anhydro compounds of the general formula

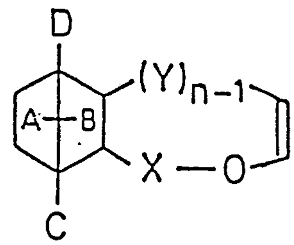

(V)

with A, B, C, D, X and Y having the same meaning as in the hydrated form and n meaning 1 or 2.

2. Chiral, enantiomerically pure compounds (R) - enantiomer respectively((S)- enantiomer) of the general formula

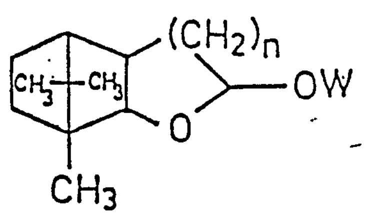

(Ia)

wherein W and n have the meaning given in claim 1 and n means 1 or 2 and respectively in case of W representing hydrogen their anhydro compounds of the general formula

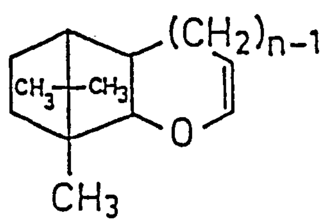

(Va)

wherein n stands for 1 or 2.

3. Chiral, enantiomerically pure (R) - enantiomera and (S) - enantiomers of the general formula

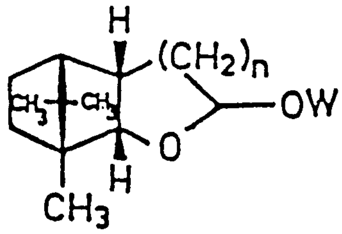

(Ib)

wherein W has the meaning given in claim 1 and n means 1 or 2 respectively in case of W representing hydrogen their anhydro compounds of the general formula

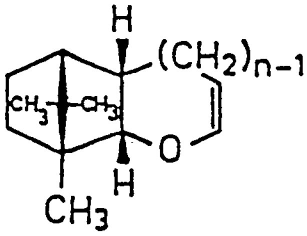

(Vb)

wherein n has the same meaning as given above.

4. (2α,3aα,4α,7α,7aα)-2,3,3a,4,5,6,7,7a-Octahydro-7,8,8-trimethyl-4,7-methanobenzofuran-2-ol (R)-enantiomer and (S)-enantiomer (Fig.1).

5. [2α(2'R*,3'aS*,4'R*,7'R*,7aS*),3aα,4α,7α,7aα]-2,2'-Oxybis[2,3,3a,4,5,6,7,7a-octahydro-7,8,8-trimethyl-4,7-methanobenzofuran] (R)-enantiomer and (S)-enantiomer (Fig.2).

6. (2α,3aα,4α,7α,7aα)-2,3,3a,4,5,6,7,7a-Octahydro-2 methoxy-7,8,8-trimethyl-4,7-methanobenzofuran (R)-enantiomer and (S)-enantiomer (Fig.3).

7. (2α,3aα,4α,7α,7aα)-2,3,3a,4,5,6,7,7a-Octahydro-2-ethoxy-7,8,8-trimethyl-4,7-methanobenzofuran (R)-enantiomer and (S)-enantiomer (Fig.4).

8. (2α,3aα,4α,7α,7aα)-2,3,3a,4,5,6,7,7a-Octahydro-2-isopropoxy-7,8,8-trimethyl-4,7-methanobenzofuran (R)-enantiomer and (S)-enantiomer (Fig.5).

9. (2α,4aα,5α,8α,8aα)-3,4,4a,5,6,7,8,8a-Octahydro-8,9,9-trimethyl-2H-5,8 methano-1-benzopyran-2-ol (R)-enantiomer and (S)-enantiomer (Fig.6).

10. [2α(2'R*,4'aS*,5'R*,8'R*,8'aS*),4aα,5α,8α, 8α]-2,2'-Oxybis[3,4,4a,5,6,7,8,8a-octahydro-8,9,9-trimethyl-2H-5,8-methano-1-benzopyran] (R)-enantiomer and (S)-enantiomer (Fig.7).

11 (2α,4aα,5α,8α,8aα)-3,4,4a,5,6,7,5,8a-Octahydro-2 methoxy-8,9,9-trimethyl-2H-5,8-methano-1-benzopyran (R)-enantiomer and (S)-enantiomer (Fig.8).

12. (3aα,4α,7α,7aα)-3a,4,5,6,7,7a-Hexahydro-7,8,8-trimethyl -4,7-methanobenzofuran (R)-enantiomer and (S)-enantionmer (Fig.9).

13. (4aα,5α,8α,8aα)-4a,5,6,7,8,8a-Hexahydro-8,9,9-trimethyl-4H-5,8-methanobenzopyran (R)-enantiomer and (S)-enantiomer (Fig.10).

14. Process for the preparation of chiral, enantiomerically pure compounds of the general formula

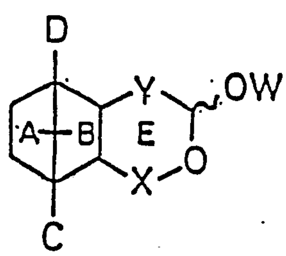

(I)

wherein a 5 or 6 membered lactol ring (E) with X and Y meaning $(CR^1R^2)_n$, with $n = 0$ to 2 and $R^1$, $R^2$ = hydrogen, lower alkyl or aryl in any combination, which doed not impair the anomeric selectivity of 1 in forming acetals, is fused in a stereospecific manner to a bicyclo[2.2.1[heptane ring system with A, B, C, D meaning H or methyl in any combination, preferably a bornan ring system and wherein W had the meaning of hydrogen, substituted or unsubstituted alkyl, cycloalkyl or the ring system itself, preferably H, the ring system itself or lower alkyl: respectively in case of W being hydrogen their anhydro compounds of the general formula

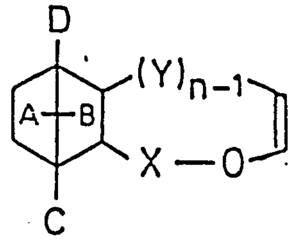

(V)

with A, B, C, D, X and Y having the same meaning as given in the hydrated form and n meaning 1 or 2 which comprises reducing a lactone of the general formula

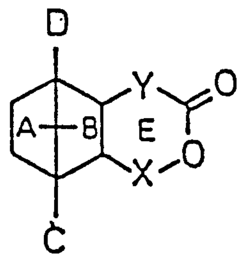

(VI)

with A, B, C, D, X and Y having the meaning given above to the lactol of the general formula I (W = H) and, if so desired, transforming this into its self condensed form (W = ring system itself) by acid catalysis in inert solvent or, if so desired, transforming this into its form I (W = substituted or unsubstituted alkyl or cycloalkyl, prererably lower alkyl) by treatment with catalytic amounts of a strong acid in the presence of an acyclic or cyclic alcohol, preferably lower alcohol or, if so desired, dehydrating this to the enolether of the general formula V by treatment with dehydrating agents preferably inorganic acid chloride/base.

15. Process according to claim 14, which comprises prefaring a chiral enantiomerically pure compound ((R)-enantiomer respectively (S)-enantiomer) of the general formula

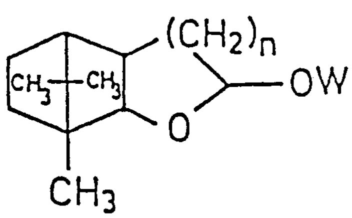

(Ia)

with W having the meaning given in claim 14 and n meaning 1 or 2 and in which the lactol ring is fused to a bornan ring system.

16. The use of chiral enantiomerically pure compounds of general formulas I respectively V, preferably of compounds or the general formulas Ia respectively Va as well as Ib respectively Vb according to claims 1 to 3 and especially the compounds according to claims 4 to 13 for protecting -OH, -SH or -NH- functional groups whereby a compound of general formula I or Ia or Ib respectively V or Va or Vb or a compound according to claims 4 to 13 is reacted under acid catalysis with a compound of the general formula

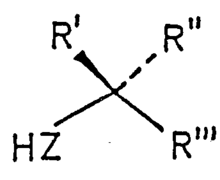

(II)

with Z meaning O, S or NH and R', R'', R''' meaning hydrogen, substituted or unsubstituted alkyl, aryl, heteroaryl, carbonyl, carboxyl or nitril groups, which are independent from or fused to each other in any combination, which does not impair the reactivity of compounds of general formula II with compounds of general formulas I respectively V, whereby in acyl compounds CR'R'' can also mean C = O, preferably hydrogen, alkyl, 1-hydroxyalkyl, 1-oxoalkyl, aryl, substituted aryl, heteroaryl, aroyl, heteroaroyl, carboxyl, alkoxycarbonyl, aryloxycarbonyl, aminocarbonyl and nitrile, whereby in case of cyclic structures R', R'' together preferably mean -$(CR^1R^2)_n$, with n = 3 to 7, and $R^1$, $R^2$ mean alkyl and aryl and as well -o-aryl-$(CR^1R^2)_n$-, with n = 2 to 4 and $R^1$, $R^2$ represent alkyl or aryl in any combination.

17. The use of chiral enantiomerically pure compounds of the general formulas I respectively V, preferably of compounds of the general formula Ia respectively Va, according to claims 1 to 3 and especially the compounds according to claims 4 to 13 for the resolution of racemates which comprises reacting a compound of the general formula I or Ia or Ib respectively V or Va or Vb or a compound according to claims 4 to 13 under acid catalyzed conditions with a racemic compound of the general formula II according to claim 16, which carries an -OH, -SH or -NH-functional group as near as possible to a chiral centre to yield a mixture of diastereomers of the general formula

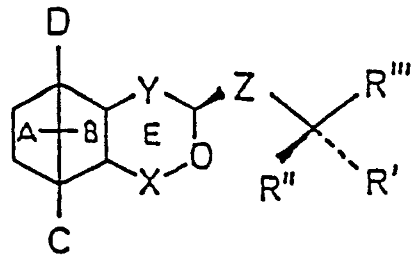

(III)

with A, B, C, D, E, X, Y, having the meaning given in claim 1 and Z, R', R'' and R''', having the meaning given in claim 16 and resolving these diastereomers, preferably by chromatography or crystallization, and then cleaving the protective group by acid catalysis, preferably methanolysis or hydrolysis.

18. The use of chiral enantiomerically pure compounds of the general formula I respectively V, preferably of compounds of the general formulas Ia resfectively Va and Ib respectively Vb according to claims 1 to 3, and especially of compounds according to claims 4 to 13 for the preparation of optically active imidoester chlorohydrates as well as optically active esters, which comprises reacting a compound of the general formula I or Ia or Ib respectively V or Va or Vb or a compound according to claims 4 to 13 with a cyanohydrine of the general formula II (Z = O, R' = CN) according to claim 16 to yield a mixture of diastereomers of the general formula III, followed by the resolution of the diastereomers and acid catalyzed reaction adding a slight excess of alcohol to yield the imidoester chlorohydrates, which may be hydrolyzed to yield the esters, if desired.

19. The use of chiral enantiomerically pure compounds of the general formula I respectively V, preferably of compounds of the general formulas Ia respectively Va and Ib respectively Vb, according to claims 1 to 3, and especially of compounds according to claims 4 to 13 for the prefaration of optically enriched alcohols via enantioselective acetal formation which comprises selective acetal formation of a racemic alcohol of the general formula II, in which the preferred meaning of Z = O, R' = H and R'' or R''' = aryl, with a compound of the general formula I or Ia or Ib respectively V or Va or Vb, or a compound according to claims 4 to 13 and subsequent cleaving of the protective group.

20. The use of chiral enantiomerically pure compounds of the general formulas I respectively V, preferably of compounds of the general formulas Ia respectively Va and Ib respectively Vb, according to claims 1 to 3 and especially of the compounds according to claims 4 to 13 for the preparation of optically active compounds containing carbonyl, carboxyl or hydroxyl groups, which comprises reacting a compound of the general formula I or Ia or Ib respectively V or Va or Vb, or a compound according to claims 4 to 13 with a prochiral compound of the general formula II which carries a carbonyl, carboxyl or nitrile group followed by the induction of chirality into the prochiral portion of the compounds of the general formula III by a stereospecific reaction and subsequently cleaving the protective group - if desired after an intermediate step of resolution of diastereomers - whereby the non chiral portion ZCR' R'' R''' preferably means $SCH_2COOR$ (R = H, alkyl, aryl) and the asymmetric induction is performed by C - alkylation after deprotonation.

**Revendications**

1. Composés chiraux, énantiomorphes purs de la formule générale

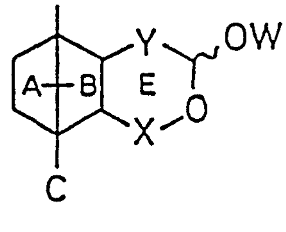

(I),

où, de préférence, un système à noyau de bornane, un noyau lactol (E) à 5 ou 6 chaînons est stéréospécifiquement condensé avec un système à noyau de bicyclo[2.2.1]heptane, formule dans laquelle A, B,

C, D représentent des atomes d'hydrogène ou des radicaux méthyle en n'importe quelle combinaison et X et Y représentent chacun un radical $(CR^1R^2)_n$, la valeur de n variant de 0 à 2 et $R^1$ et $R^2$ représentant des atomes d'hydrogène, des radicaux alkyle inférieurs ou aryle en n'importe quelle combinaison, pour autant que la sélectivité anomère ne soit pas fâcheusement influencée au cours de réactions d'acétalisation, et W représente un atome d'hydrogène, un radical cycloalkyle, alkyle substitué ou non substitué, ou le système cyclique lui-même plus particulièrement un atome d'hydrogène, le système cyclique lui-même ou un radical alkyle inférieur, ou bien, dans le cas où W représente un atome d'hydrogène, les anhydrocomposés de la formule générale

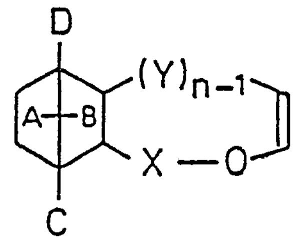

(V),

dans laquelle A, B, C, D, X et Y possèdent des mêmes significations que dans la forme hydratée et n est égal à 1 ou à 2.

2. Composés chiraux, énantiomorphes purs (énantiomorphe (R) ou énantiomorphe (S)) de la formule générale

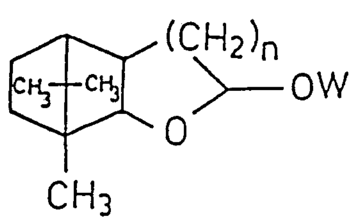

(I a),

dans laquelle W et n possèdent les significations qui leur ont été attribuées plus haut dans la revendication 1 et n est égal à 1 ou à 2, ou dans le cas où W représente un atome d'hydrogène, leurs anhydro-composés de la formule générale

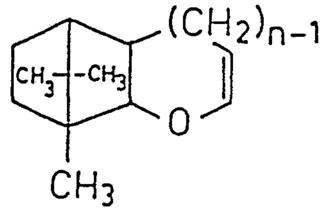

(Va),

dans laquelle n est égal à 1 ou à 2.

3. Enantiomorphes (R) et énantiomorphes (S) chiraux, énantiomorphes purs, de la formule générale:

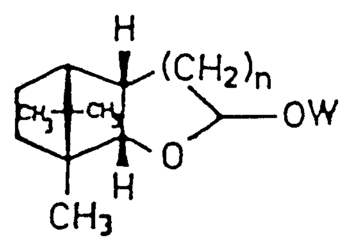

(Ib),

dans laquelle W possède les mêmes significations que celles qui lui ont été attribuées dans la revendication 1 et n est égal à 1 ou à 2, ou dans le cas où W représente un atome d'hydrogène, leurs anhydro-composés de la formule générale

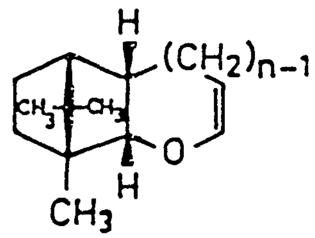

(Vb),

dans laquelle N possède les significations qui lui ont été précédemment attribuées.

4. (2α,3aα,4α,7α,7aα)-2,3,3a,4,5,6,7,7a-octahydro-7,8,8-triméthyl-4,7-méthanobenzofuran-2-ol énantiomorphe (R) et énantiomorphe (S) (Fig. 1).

5. (2α(2'R*,3'aS*,4R*,7'R*,7'aS*),3aα,4α,7α,7aα)-2,2'-Oxy-bis-[2,3,3a,4,5,6,7,7a-octahydro-7,8,8-triméthyl-4,7-méthanobenzofurane] énantiomorphe (R) et énantiomorphe(S) (Fig. 2).

6. (2α,3aα,4α,7α,7aα)-2,3,3a,4,5,6,7,7a-Octahydro-2-méthoxy-7,8,8-triméthyl-4,7-méthanobenzofurane énantiomorphe (R) et énantiomorphe (S) (Fig. 3).

7. (2α,3aα,4α,7α,7aα)-2,3,3a,4,5,6,7,7a-Octahydro-2-éthoxy-7,8,8-triméthyl-4,7-méthanobenzofurane énantiomorphe (R) et énantiomorphe (S) (Fig. 4).

8. (2α,3aα,4α,7α,7aα)-2,3,3a,4,5,6,7,7a-Octahydro-2-isopropoxy-7,8,8-triméthyl-4,7-méthanobenzofurane énantiomorphe (R) et énantiomorphe (S) (Fig. 5).

9. (2α,4aα,5α,8α,8aα)-3,4,4a,5,6,7,8,8a-Octahydro-8,9,9-triméthyl-2H-5,8-méthano-1-benzopyran-2-ol énantiomorphe (R) et énantiomorphe (S) (Fig. 6).

10. (2α(2'R*,4'aS*,5'R*,8'R*,8'aS*),4aα,5α,8α,8aα)-2,2-Oxybis[3,4,4a,5,6,7,8,8a-octahydro-8,9,9-triméthyl-2H-5,8-méthano-1-benzopyrane] énantiomorphe (R) et énantiomorphe (S) (Fig. 7).

11. (2α,4aα,5α,8α,8aα)-3,4,4a,5,6,7,8,8a-Octahydro-2-méthoxy-8,9,9-triméthyl-2H-5,8-méthano-1-benzopyrane énantiomorphe (R) et énantiomorphe (S) (Fig. 8).

12. (3aα,4α,7α,7aα)-3a,4,5,6,7,7a-hexahydro-7,8,8-triméthyl-4,7-méthanobenzofurane énantiomorphe (R) et énantiomorphe (S) (Fig. 9).

13. (4aα,5α,8α,8aα)-4a,5,6,7,8,8a-Hexahydro-8,9,9-triméthyl-4H-5,8-méthanobenzopyrane énantiomorphe (R) et énantiomorphe (S) (Fig. 10).

14. Procédé de préparation de composés chiraux, énantiomorphes purs, de la formule générale

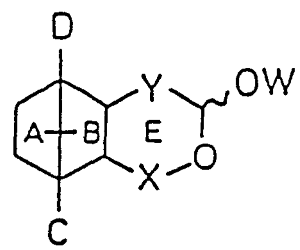

(I),

où de préférence un système à noyau de bornane, un noyau lactol (E) à 5 ou 6 chaînons est stéréospécifiquement condensé avec un système à noyau de bicyclo[2.2.1]heptane, formule dans laquelle A, B, C, D représentent des atomes d'hydrogène ou des radicaux méthyle en n'importe quelle combinaison et X et Y représentent chacun un radical $(CR^1R^2)_n$, la valeur de n variant de 0 à 2 et $R^1$ et $R^2$ représentant des atomes d'hydrogène, des radicaux alkyle inférieurs ou aryle en n'importe quelle combinaison, pour autant que la sélectivité anomère ne soit pas fâcheusement influencée au cours de réactions d'acétalisation, et W représente un atome d'hydrogène, un radical cycloalkyle, alkyle substitué ou non substitué, ou le système cyclique lui-même, plus particulièrement un atome d'hydrogène, le système cyclique lui-même ou un radical alkyle inférieur, ou bien, dans le cas où W représente un atome d'hydrogène, les anhydrocomposés de la formule générale

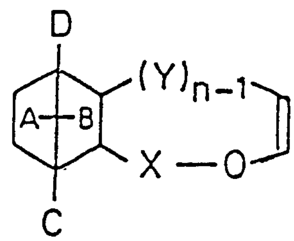

(V),

dans laquelle A B, C, D, X et Y possèdent les mêmes significations que dans la forme hydratée et n est égal à 1 ou à 2, caractérisé en ce que l'on réduit une lactone de la formule générale

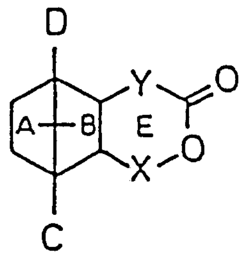

(VI),

dans laquelle A, B, C, D, X et Y possèdent les significations qui leur ont été précédemment attribuées, en un lactol de la formule générale I (W = H) et on transforme éventuellement celui-ci par catalyse acide, dans un solvant inerte en sa forme autocondensée (W = système cyclique lui-même),

ou bien on transforme éventuellement celui-ci, en présence d'un alcool acyclique ou cyclique, de préférence un alcool inférieur, en composés de la formule générale I dans laquelle W représente un radical cycloalkyle ou alkyle substitué ou non substitué, de préférence un radical alkyle inférieur,

ou bien on déshydrate éventuellement celui-ci par traitement à l'aide d'agents déshydratants, de préférence un chlorure d'acide inorganique/base, de façon à obtenir un éther énolique de la formule V.

15. Procédé suivant la revendication 14, caractérisé en ce, qu'un composé chiral, énantiomorphe pur (énantiomorphe (R) ou énantiomorphe (S)) de la formule générale

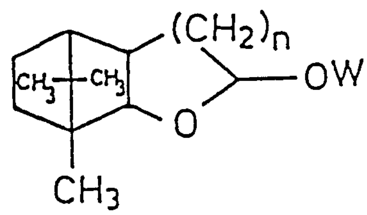

(Ia),

dans laquelle W possède la signification qui lui a été attribuée dans la revendication 14 et n est égal à 1 ou à 2 et dans laquelle le noyau lactol est condensé avec we ossature bornanique, est préparé.

16. Utilisation de composés chiraux, énantiomorphes purs, des formules générales I et V, de préférence de composés des formules générales Ia et Va, comme aussi Ib et Vb, suivant les revendications 1 à 3 et, plus particulièrement, des composés suivant les revendications 4 à 13, pour la protection de radicaux -OH, -SH, -NH fonctionnels, conformément à laquelle on fait réagir un composé de la formule I ou Ia ou Ib ou V ou Va ou Vb ou un composé suivant les revendications 4 à 13, sous catalyse à l'acide, sur un composé de la formule générale

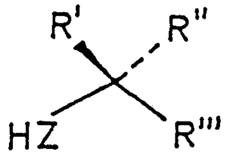

(II),

dans laquelle Z = O, S ou NH et les symboles R', R'' et R''' représentent des atomes d'hydrogène ou des radicaux nitrile, carboxyle, cabonyle, hétéroaryle, aryle, alkyle substitués ou non substitués, indépendants les uns des autres ou mutuellement liés, en n'importe quelles combinaisons, pour autant que ceux-ci ne limitent pas la réactivité de composés de la formule générale II avec des composés de la formule générale I ou V, où dans des composes acyles CR'R'' représente également un groupe

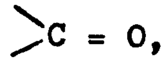

en particulier, de l'hydrogène, des radicaux alkyle, 1-hydroxyalkyle, 1-oxoalkyle, aryle, aryle substitué, hétéroaryle, aroyle, hétéroaroyle, carboxyle, alcoxycarbonyle, aryloxycarbonyle, aminocarbonyle, et nitrile, où, pour des structures cycliques, R' et R'' ensemble représentent, de préférence un radical $-(CR^1R^2)_n-$ das lequel n a une valeur qui varie de 3 à 7 et $R^1$ et $R^2$ représentent des radicaux alkyle et aryle, comme aussi des radicaux $-O-aryl-(CR^1R^2)_n-$ où n a une valeur qui varie de 2 à 4 et $R^1$ et $R^2$ représentent des radicaux alkyle ou aryle en n'importe quelle combinaison.

17. Utilisation de composés chiraux, énantiomorphes purs, des formules générales I et V, de préférence, de composés des formules générales Ia et Va, comme aussi Ib et Vb suivant les revendications 1 à 3 et, plus

particulièrement, des composés suivant les revendications 4 à 13, pour la séparation des racémiques, suivant laquelle on fait réagir un composé de la formule générale I ou Ia ou Ib ou V ou Va ou Vb ou un composé suivant les revendications 4 à 13, sur un racémique de la formule générale II suivant la revendication 16, qui présente une fonction -OH, -SH, ou -NH, si possible au voisinage d'un centre chiral, sous catalyse à l'acide, de manière à obtenir un mélange des diastéréoisomères de la formule générale

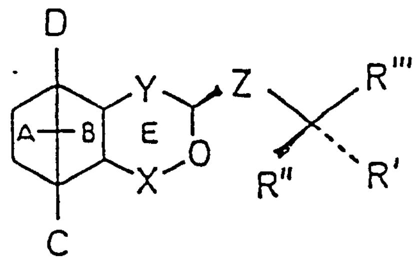

(III) ,

dans laquelle A, B, C, D, E, X et Y possèdent les mêmes significations que celles qui leur ont été attribuées dans la revendication 1 et Z, R', R'' et R''' possèdent les mêmes significations que celles qui leur ont été attribuées dans la revendication 16 et on sépare ces diastéréoisomères, de préference par chromatographie ou cristallisation, puis on élimine les radicaux protecteurs, de nouveau sous catalyse à l'acide, de préference méthanolyse ou hydrolyse.

18. Utilisation de composés chiraux, énantiomorphes purs, des formules générales I et V, de préference de composés des formules générales Ia et Va, comme aussi Ib et Vb suivant les revendications 1 à 3 et, plus particulièrement, des composés suivant les revendications 4 à 13, en vue de la préparation de chlorhydrates d'imidoesters optiquement actifs, comme aussi d'esters optiquement actifs, suivant laquelle on fait réagir un composé de la formule générale I ou Ia ou Ib ou V ou Va ou Vb ou un composé suivant les revendications 4 à 13, sur une cyanhydrine de la formule générale II (Z = O, R' = CN) suivant la revendication 16, de façon à obtenir un mélange des diastéréoisomères de la formule générale III, puis on obtient les chlorhydrates d'imidoesters après séparation des diastéréoisomères sous addition d'un faible excès d'un alcool et sous catalyse à l'acide, chlorhydrates d'imidoesters que l'on saponifie éventuellement en les esters optiquement actifs.

19. Utilisation de composés chiraux, énantiomorphes purs, des formules générales I et V, de préference de composés des formules générales Ia et Va, comme aussi Ib et Vb, suivant les revendications 1 à 3 et, plus particulièrement, des composés suivant les revendications 4 à 13, en vue de l'obtention d'alcools optiquement enrichis par acétalisation énantio-sélective, suivant laquelle on acétalise sélectivement le racémique d'un alcool de la formule générale II dans laquelle, de préference, Z = O, R' = H et R'' ou R''' = aryle, avec un composé de la formule I ou Ia ou Ib ou V ou Va ou Vb ou un composé suivant les revendications 4 à 13 et on sépare ensuite le groupe protecteur.

20. Utilisation de composés chiraux, énantiomorphes purs, des formules générales I et V, de préference, de composés des formules générales Ia et Va, comme aussi Ib et Vb, suivant les revendications 1 à 3 et, plus particulièrement, des composés suivant les revendications 4 à 13, en vue de la préparation de composés carbonylés, carboxylés ou hydroxylés, optiquement actifs, suivant laquelle on fait réagir un composé de la formule générale I ou Ia ou Ib ou V ou Va ou Vb ou un composé suivant les revendications 4 à 13, sur un composé prochiral de la formule générale II, qui porte une fonction carbonyle, carboxyle ou nitrile, on induit une asymétrie dans le reste prochiral des composés obtenus de la formule générale III par une réaction stéréospécifique et on sépare ultérieurement les radicaux protecteurs, éventuellement après une étape de séparation des diastéréoisomères, où le reste chiral -ZCR'R''R''' représente, de préférence, le radical $SCH_2COOR$ (R = H, alkyle, aryle) et on fait suivre l'induction asymétrique, de préférence, d'une C-alkylation après déprotonation.

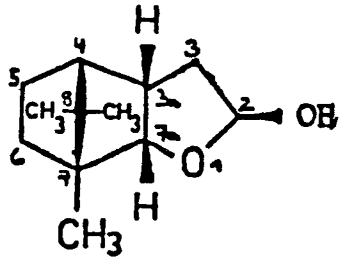

Fig.1

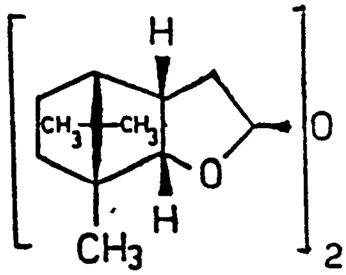

Fig.2

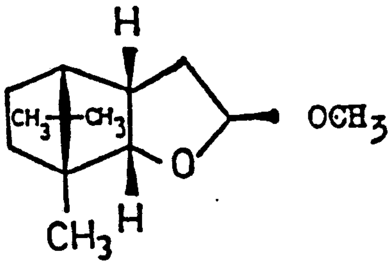

Fig.3

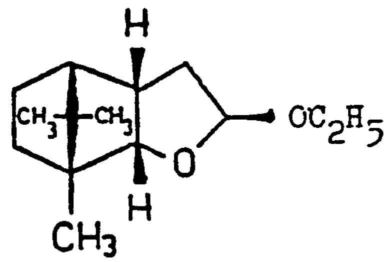

Fig.4

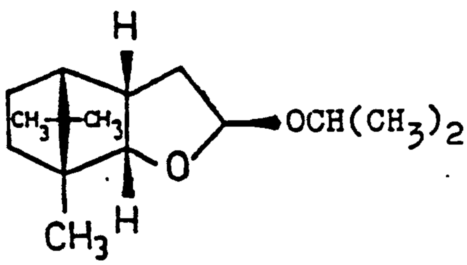

Fig.5

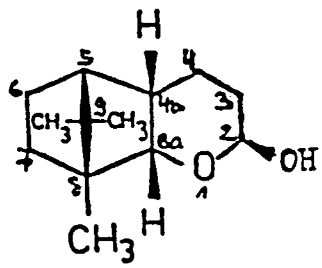

Fig.6

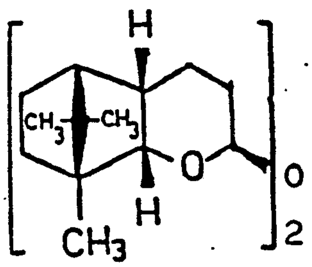

Fig.7

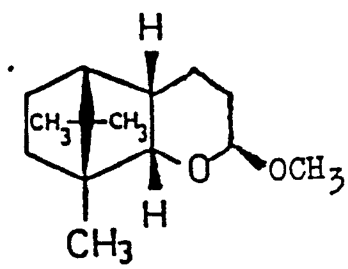

Fig.8

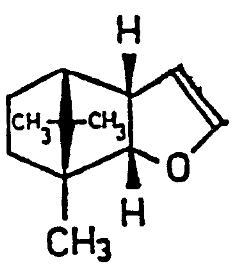

Fig.9

und Spiegelbilder

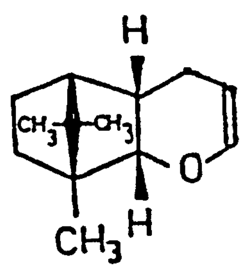

Fig.10